(19) Europäisches Patentamt
European Patent Office
Office européen des brevets

(11) Veröffentlichungsnummer: **0 307 886 B1**

(12) **EUROPÄISCHE PATENTSCHRIFT**

(45) Veröffentlichungstag der Patentschrift: **25.11.92**

(51) Int. Cl.⁵: **C10M 135/36**, C07D 277/16, C07D 263/16, C07D 279/04

(21) Anmeldenummer: **88115017.1**

(22) Anmeldetag: **14.09.88**

# (54) Aminomethylderivate von Mono- und Dithiocarbamaten als Schmiermitteladditive.

(30) Priorität: **17.09.87 CH 3589/87**

(43) Veröffentlichungstag der Anmeldung:
**22.03.89 Patentblatt 89/12**

(45) Bekanntmachung des Hinweises auf die Patenterteilung:
**25.11.92 Patentblatt 92/48**

(84) Benannte Vertragsstaaten:
**BE DE ES FR GB IT NL**

(56) Entgegenhaltungen:
**EP-A- 0 203 033**
**DE-A- 2 558 398**
**US-A- 3 068 098**
**US-A- 4 543 318**

**CHEMICAL ABSTRACTS, Band 43, Nr. 21, 10. November 1949, Seite 8514, Zusammenfassung Nr. 8514 f-g, Columbus, Ohio, US; D.A. McGINTY et al.: "Comparative activities of thiouracil and other antithyroid compounds in the rhesus monkey", & ENDOCRINOLOGY 44, 546-54 (1949)**

**CHEMICAL ABSTRACTS, Band 94, Nr. 10, 9. März 1981, Seite 622, Zusammenfassung Nr. 74729g, Columbus, Ohio, US;**

(73) Patentinhaber: **CIBA-GEIGY AG**
**Klybeckstrasse 141**
**CH-4002 Basel(CH)**

(72) Erfinder: **Camenzind, Hugo, Dr.**
**Av. Général Guisan 42**
**CH-1700 Fribourg(CH)**

(74) Vertreter: **Zumstein, Fritz, Dr. et al**
**Bräuhausstrasse 4**
**W-8000 München 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft Schmiermittel- oder Hydraulikölzusammensetzungen enthaltend öllösliche Derivate von Mono- und Dithiocarbamaten, die Verwendung solcher Derivate als Schmiermitteladditive, sowie die Verbindungen selbst, soweit sie neu sind.

Mineralischen und synthetischen Schmiermitteln werden üblicherweise Additive zur Verbesserung der Gebrauchseigenschaften zugesetzt. Zur Verbesserung der Verschleissschutzeigenschaften werden den Schmiermitteln Hochdruck- und verschleissmindernde Additive zugefügt. An diese wird die Anforderung gestellt, dass sie nicht korrodierend auf die zu schmierenden Metallteile wirken und eine gute Hitzebeständigkeit aufweisen.

Hierfür werden heute vorzugsweise phosphor- und schwefelhaltige Verbindungen verwendet wie z.B. Dialkyldithiophosphate gemäss der DE-A-2,921,620. Im Hinblick auf die Verwendung von Katalysatoren im Abgassystem von Verbrennungsmotoren soll jedoch der Phosphorgehalt von Schmierölen möglichst tief gehalten werden, damit die Katalysatoren nicht desaktiviert werden (H.S. Gandhi et al., Applied Catalysis 3 - (1982), 79-88).

Phosphorfreie Additive für Schmiermittel wie z.B. Aminomethylderivate von Benzothiazolinthion werden in der EP-A 203,033 oder wie z.B. N,N-disubstituierte S-Thiiranylmethylcarbamothioate in der EP-A 211,806 beschrieben.

Ferner wird in der DE-A 2,701,215 ein Verfahren zur Herstellung von Thiazolin-2-thionen und deren Verwendung als Vulkanisationsbeschleuniger für Polychloroprenkautschuke beschrieben. F. Fülöp et al. beschreiben in Synthesis (1985) 1149-51 eine Methode zur Darstellung von 2-Thioxotetrahydro-1,3-oxazol und 2-Thioxotetrahydro-1,3-oxazin.

Weiter sind aus den US-A 3,068,098 sowie US-A 4,543,318 Aminomethyl-derivate von Thiazolin-2-thion und Oxazolin-2-thion als Haftvermittler für Emulsionen auf Fotopapier sowie als Haftvermittler für Fotoresists auf Kupferunterlagen bekannt.

Es wurde nun gefunden, dass öllösliche Aminomethylderivate von cyclischen Mono- und Dithiocarbamaten in mineralischen und synthetischen Schmiermitteln ausgezeichnete Eigenschaften hinsichtlich Schutz vor Reibungsabnutzung, Lasttragevermögen, Schutz der Metallteile vor Korrosion und Aschefreiheit aufweisen.

Die vorliegende Erfindung betrifft daher eine Zusammensetzung enthaltend ein oder mehrere Schmiermittel oder Hydrauliköle auf der Basis von Mineralöl, synthetischen Oelen oder Gemischen davon und mindestens eine Verbindung der Formeln I, II und/oder III,

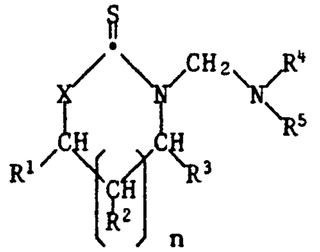

I

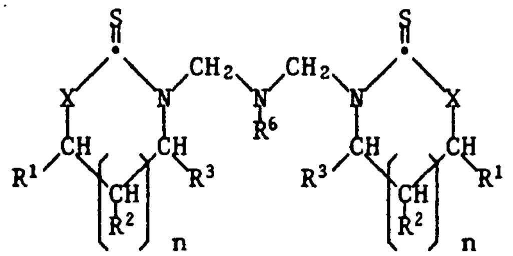

II

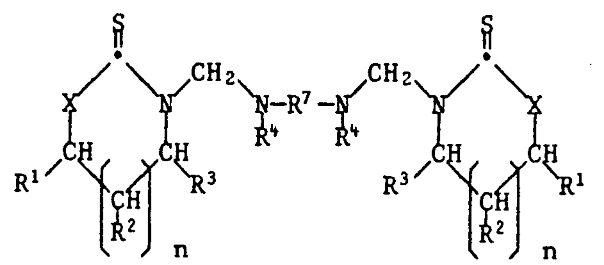

III

worin

$R^1$, $R^2$, $R^3$, $R^4$, $R^5$ und $R^6$ unabhängig voneinander $C_1$-$C_{24}$-Alkyl oder $C_1$-$C_{24}$-Alkyl das durch Oxo- oder Thionogruppen substituiert ist oder $C_3$-$C_{24}$-Alkyl, das durch

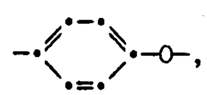

-O-, -S- und/oder -N($R^8$)-, mit $R^8$ als Wasserstoff oder $C_1$-$C_{12}$-Alkyl, unterbrochen ist oder $C_3$-$C_{24}$-Alkyl, das durch

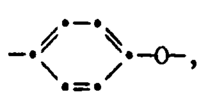

-O-, -S- und/oder -N($R^8$)-, mit $R^8$ als Wasserstoff oder $C_1$-$C_{12}$-Alkyl, unterbrochen ist und das durch Oxo- oder Thionogruppen substituiert ist, oder $C_2$-$C_{24}$-Alkenyl, oder unsubstituiertes oder durch ein oder zwei $C_1$-$C_{12}$-Alkyl-, $C_1$-$C_4$-Alkoxy-, $C_2$-$C_{24}$-Carboalkoxy- oder Nitrogruppen substituiertes Phenyl oder Naphtyl, oder $C_7$-$C_{10}$-Phenylalkyl bedeuten und $R^1$, $R^2$, $R^3$ und $R^4$ zusätzlich Wasserstoff bedeutet, oder $R^1$ und $R^2$ bzw. $R^1$ und $R^3$ im Fall von n gleich Null, zusammen mit den C-Atomen, an die sie gebunden sind, einen 5- oder 6-gliedrigen aliphatischen Ring bilden oder einen 5- oder 6-gliedrigen aliphatischen Ring, der durch Oxo- oder Thionogruppen substituiert ist, bilden oder einen 5- oder 6-gliedrigen aliphatischen Ring bilden, der durch -N($R^8$)-, mit $R^8$ in der vorstehenden Bedeutung, -O-oder -S- unterbrochen ist oder einen 5- oder 6-gliedrigen aliphatischen Ring bilden, der durch -N($R^8$)-, mit $R^8$ in der vorstehenden Bedeutung, -O-oder -S-unterbrochen und durch Oxo- oder Thionogruppen substituiert ist, oder worin $R^4$ und $R^5$ zusammen mit dem N-Atom, an das sie gebunden sind, einen 5- bis 7-gliedrigen aliphatischen-heterocyclischen Ring bilden, oder worin $R^4$ und $R^5$ zusammen mit dem N-Atom, an das sie gebunden sind, einen 5- bis 7-gliedrigen aliphatischen-heterocyclischen Ring bilden und der Ring durch Oxo- oder Thionogruppen substituiert ist und/oder zusätzlich zu dem N-Atom noch weitere Heteroatome [N($R^8$), O, S] enthält, und worin $R^7$ $C_2$-$C_{12}$-Alkylen, $C_2$-$C_{12}$-Alkylen, das durch -N($R^8$)-, -O-, und/oder -S-unterbrochen ist, $C_2$-$C_{12}$-Alkylen, das Oxo- oder Thionogruppen enthält oder $C_2$-$C_{12}$-Alkylen, das durch -N($R^8$)-, -O- und/oder -S-unterbrochen ist und das Oxo- oder Thionogruppen enthält, $C_6$-$C_{15}$-Cycloalkylen, $C_6$-$C_{15}$-Arylen, Carbonyl oder 'Thiocarbonyl bedeutet oder die Gruppe -N($R^4$)-$R^7$-N($R^4$)- einen Piperazin-1,4-diylrest oder einen Piperazin-1,4-diylrest, der durch eine oder mehrere Methylgruppen substituiert ist, darstellt, und X Sauerstoff oder Schwefel und n die Zahl Null oder 1 bedeuten.

Stellen $R^1$, $R^2$, $R^3$, $R^4$, $R^5$ und $R^6$ $C_1$-$C_{24}$-Alkyl dar, so handelt es sich um geradkettige oder verzweigte Alkylreste wie beispielsweise Methyl, Ethyl, n-Propyl, Isopropyl, sec.-Butyl, tert.-Butyl, geradkettiges oder verzweigtes Pentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Eicosyl, Heneicosyl, Docosyl, Tricosyl und Tetracosyl.

Stellen $R^1$, $R^2$, $R^3$, $R^4$, $R^5$ und $R^6$ $C_3$-$C_{24}$-Alkyl dar, das durch

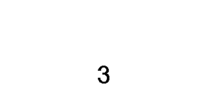

-O-, -S- oder -N($R^8$)- unterbrochen ist, so kann sich das Heteroatom bzw. die

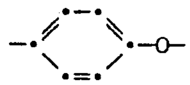

Gruppe in jeder möglichen Position befinden, und der $C_1$-$C_{24}$-Alkylrest kann einfach oder mehrfach unterbrochen sein, wobei die Unterbrechung sowohl durch gleiche oder verschiedene Heteroatome als auch durch

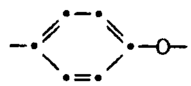

Gruppen erfolgen kann. Bevorzugt ist jedoch eine Unterbrechung. Die Zahl der C-Atome steht für die gesamte Anzahl der Glieder im Rest.

Stellen $R^1$, $R^2$, $R^3$, $R^4$, $R^5$ und $R^6$ durch Oxo- oder Thionogruppen substituiertes $C_1$-$C_{24}$-Alkyl dar, so kann der Alkylrest ein- oder mehrfach in jeder möglichen Position substituiert sein, wobei gleiche oder verschiedene Substituenten möglich sind. Insbesondere ist ein Oxo-Substituent an einem C-Atom möglich, das sich unmittelbar neben einer O-Unterbrechung der Alkylkette befindet, sodass eine C(O)-O- Gruppe gebildet wird.

Stellen $R^1$, $R^2$, $R^3$, $R^4$, $R^5$ und $R^6$ $C_2$-$C_{24}$-Alkenyl dar, so handelt es sich um geradkettige oder verzweigte Alkenylreste, die eine oder mehrere, bevorzugt jedoch eine Doppelbindung enthalten, wie beispielsweise Vinyl, Allyl, n-Butenyl, 1,3-Butadienyl, i-Pentenyl, Pentenyl, Hexenyl, Heptenyl, Octenyl, Nonenyl, Decenyl, Undecenyl, Dodecenyl, Tridecenyl, 2-Nonyl-2-butenyl, Tetradecenyl, Pentadecenyl, Hexadecenyl und 8-Heptadecenyl, 2-Octadecenyl, Oleyl, Nonadecenyl, Eicosenyl, Heneicosenyl, Docosenyl, Tricosenyl und Tetracosenyl. Bevorzugt sind Allyl und Oleyl.

Stellen $R^1$, $R^2$, $R^3$, $R^4$, $R^5$ und $R^6$ durch $C_1$-$C_{12}$-Alkyl substituiertes Phenyl bzw. Naphthyl dar, so können der Phenyl- bzw. Naphthylrest ein-oder zweifach, bevorzugt jedoch einfach, substituiert sein; bei $C_1$-$C_{12}$-Alkyl handelt es sich beispielsweise um Methyl, Ethyl, n-Propyl, Isopropyl, n-Butyl, Isobutyl, sec.-Butyl, tert.-Butyl, geradkettiges oder verzweigtes Octyl, Nonyl oder Dodecyl.

Stellen $R^1$, $R^2$, $R^3$, $R^4$, $R^5$ und $R^6$ durch $C_1$-$C_4$-Alkoxy substituiertes Phenyl bzw. Naphthyl dar, so können der Phenyl- bzw. Naphthylrest ein-oder zweifach, bevorzugt jedoch einfach, substituiert sein; bei $C_1$-$C_4$-Alkoxy handelt es sich beispielsweise um Methoxy, Ethoxy, n-Propoxy, Isopropoxy, n-Butoxy, Isobutoxy, sec.-Butoxy oder tert.-Butoxy.

Stellen $R^1$, $R^2$, $R^3$, $R^4$, $R^5$ und $R^6$ durch $C_2$-$C_{24}$-Carboalkoxy substituiertes Phenyl bzw. Naphthyl dar, so können der Phenyl- bzw. Naphthylrest ein- oder zweifach, bevorzugt jedoch einfach substituiert sein; $C_2$-$C_{24}$-Carboalkoxy enthält 1-23 Kohlenstoffatome im Alkylteil und kann z.B. Carbomethoxy, Carboethoxy, Carbopropoxy oder Carbo-2-ethylhexyloxy sein.

Stellen $R^1$, $R^2$, $R^3$, $R^4$, $R^5$ und $R^6$ durch Nitro substituiertes Phenyl bzw. Naphthyl dar, so können der Phenyl- bzw. Naphthylrest ein- oder zweifach, bevorzugt jedoch einfach, substituiert sein.

Stellen $R^1$, $R^2$, $R^3$, $R^4$, $R^5$ und $R^6$ $C_7$-$C_{10}$-Phenylalkyl dar, so handelt es sich beispielsweise um Benzyl, 1- oder 2-Phenethyl, 3-Phenylpropyl, $\alpha$,$\alpha$-Dimethylbenzyl oder 2-Phenylisopropyl, vorzugsweise jedoch um Benzyl.

Stellen $R^1$ und $R^2$ oder $R^1$ und $R^3$ zusammen mit den C-Atomen, an die sie gebunden sind, einen 5- oder 6-gliedrigen aliphatischen Ring dar, der durch -N($R^8$)-, -O- oder S unterbrochen ist und/oder durch Oxo- oder Thionogruppen substituiert ist, so bilden $R^1$ und $R^2$ oder $R^1$ und $R^3$ zusammen beispielsweise die Gruppen 2-Oxo-but-1,4-diyl, 2-Oxa-but-1,4-diyl, 2-Thia-but-1,4-diyl, 2-Thiono-prop-1,3-diyl, 2-(Methylaza)-but-1,4-diyl oder 2-Oxo-3-oxa-but-1,4-diyl.

Stellen $R^4$ und $R^5$ zusammen mit dem N-Atom, an das sie gebunden sind, einen 5- bis 7-gliedrigen aliphatisch-heterocyclischen Ring dar, der zusätzlich zu dem N-Atom noch weitere Heteroatome [N($R^8$), O, S] enthält und/oder durch Oxo- oder Thionogruppen substituiert ist, so bilden sie beispielsweise einen Piperidin-, Perhydroazepin-, Piperazin-, 4-Methylpiperazin-, Morpholin-, Piperidin-2-on- oder Piperidin-2-thionring. Bevorzugt ist ein Piperidin-, Perhydroazepin- oder Morpholinring.

Stellt $R^7$ $C_2$-$C_{12}$-Alkylen dar, so handelt es sich um geradkettige oder verzweigte Alkylenreste, wie beispielsweise Ethylen, Propylen, Tri-methylen, Tetramethylen, 2,2-Dimethyl-1,3-trimethylen, Hexamethylen, Heptamethylen, Octamethylen oder Dodecamethylen.

Stellt $R^7$ durch -N($R^8$)-, -O- und/oder -S- unterbrochenes $C_2$-$C_{12}$-Alkylen dar, kann dieses einfach oder mehrfach, bevorzugt jedoch einfach, unterbrochen sein wie beispielsweise 2-Oxa-1,3-propylen, 2,4-Di-oxa-1,5-pentylen, 3,5-Di-oxa-1,6-hexylen, 3-Oxa-1,6-hexylen, 2,5,8-Tri-oxa-1,9-nonylen, 2,5,8-Tri-oxa-1,11-undecylen, 2-Thia-1,3-propylen, 2,4-Di-thia-1,5-pentylen, 2,5,8-Trithia-1,11-undecylen, 3-(Methylaza)-1,5-pentylen oder 3,6-Di-(methylaza)-1,11-undecylen.

Stellt $R^7$ $C_6$-$C_{15}$-Cycloalkylen dar, so handelt es sich beispielsweise um Cyclo-1,4-hexylen, Cyclo-1,5-octylen, Cyclo-1,4-dodecylen oder Cyclo-1,5-pentadecylen.

Stellt $R^7$ $C_6$-$C_{15}$-Arylen dar, so handelt es sich beispielsweise um 1,4-Phenylen, 1,3-Phenylen, 2,3-Naphthylen, 1,5-Naphthylen, 1,8-Naphthylen, 9,10-Anthracenylen, 9,10-Phenanthrenylen oder 4,4'-Biphenylylen. Bevorzugt sind 1,4-Phenylen, 2,3-Naphthylen und 4,4'-Biphenylylen, insbesondere 4,4'Biphenylylen.

Stellt $R^8$ $C_1$-$C_{12}$-Alkyl dar, so handelt es sich um geradkettige oder verzweigte Alkylreste, wie beispielsweise Methyl, Ethyl, n-Propyl, Isopropyl, n-Butyl, Isobutyl, sec.-Butyl, tert.-Butyl, geradkettiges oder verzweigtes Pentyl, Hexyl, Heptyl, Octyl, Nonyl, Decyl, Undecyl oder Dodecyl. Bevorzugt ist Methyl, Ethyl, n-Propyl, Isopropyl, n-Butyl, Isobutyl, sec.-Butyl und tert.-Butyl, insbesondere Methyl.

Bevorzugt ist eine Zusammensetzung, worin in den Verbindungen der Formeln I, II oder III n gleich 1 ist.

Besonders bevorzugt ist eine Zusammensetzung, worin in den Verbindungen der Formeln I, II oder III $R^2$ Wasserstoff, $C_1$-$C_{12}$-Alkyl, Phenyl oder Benzyl bedeutet.

Speziell bevorzugt ist eine Zusammensetzung, worin in den Verbindungen der Formeln I, II oder III $R^1$ und $R^2$ zusammen mit den C-Atomen, an die sie gebunden sind, einen 6-gliedrigen aliphatischen Ring bilden.

Ebenfalls bevorzugt ist eine Zusammensetzung, worin in den Verbindungen der Formeln I, II oder III n gleich Null ist.

Weiter bevorzugt ist eine Zusammensetzung, worin in den Verbindungen der Formeln I, II oder III $R^1$ Wasserstoff, $C_1$-$C_{12}$-Alkyl, das gegebenenfalls durch eine Oxogruppe substituiert ist, $C_3$-$C_{12}$-Alkyl das durch

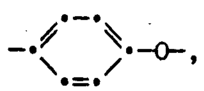

-O-, -S- unterbrochen und gegebenenfalls durch eine Oxogruppe substituiert ist, Phenyl oder Benzyl bedeutet.

Insbesondere bevorzugt ist eine Zusammensetzung, worin in den Verbindungen der Formeln I, II oder III $R^3$ Wasserstoff oder $C_1$-$C_4$-Alkyl, bevorzugt jedoch Wasserstoff, bedeutet.

Ganz speziell bevorzugt ist eine Zusammensetzung, worin in den Verbindungen in Formeln I, II oder III $R^1$ und $R^3$ Wassertoff bedeuten.

Ebenfalls von Interesse ist eine Zusammensetzung, worin in den Verbindungen der Formeln I oder III $R^4$ Wasserstoff, $C_1$-$C_{12}$-Alkyl, $C_3$-$C_{18}$-Alkenyl, Phenyl oder Benzyl, insbesondere jedoch Wasserstoff oder $C_1$-$C_8$-Alkyl, bedeutet.

Weiter von Interesse ist eine Zusammensetzung, worin in der Verbindung der Formel I $R^5$ $C_1$-$C_{18}$-Alkyl, $C_3$-$C_{18}$-Alkenyl, Phenyl oder Benzyl, insbesondere jedoch $C_4$-$C_{18}$-Alkyl, Oleyl oder Phenyl, bedeutet.

Besonders interessant ist eine Zusammensetzung, worin in der Verbindung der Formel I $R^4$ und $R^5$ zusammen mit dem N-Atom, an das sie gebunden sind einen Piperidin-, Perhydroazepin-, Morpholin-, Piperazin- oder einen 4-N-Methylpiperazinring, insbesondere jedoch einen Piperidin-oder Morpholinring, bilden.

Weiter interessant ist eine Zusammensetzung, worin in der Verbindung der Formel II $R^6$ $C_1$-$C_{12}$-Alkyl, $C_3$-$C_{18}$-Alkenyl, Phenyl oder Benzyl, insbesondere jedoch Oleyl oder Benzyl und ganz besonders Oleyl, bedeutet.

Zusätzlich von Interesse ist eine Zusammensetzung, worin in der Verbindung der Formel III $R^7$ 4,4'-Biphenylylen bedeutet.

Weiter von Interesse ist eine Zusammensetzung, worin in der Verbindung der Formel III die Gruppe -N-($R^4$)-$R^7$-N($R^4$)- Piperazin-1,4-diyl bedeutet.

Von speziellem Interesse ist eine Zusammensetzung, worin in den Verbindungen der Formeln I, II oder III X Schwefel bedeutet.

Ganz besonders bevorzugt werden Zusammensetzungen, enthaltend mindestens eine Verbindung der Formeln

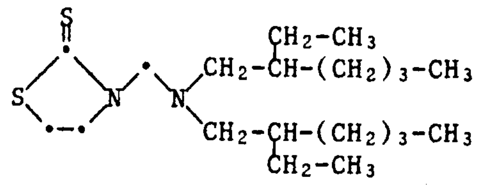 , 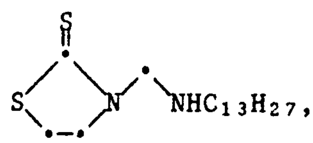

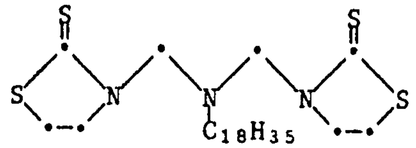 , 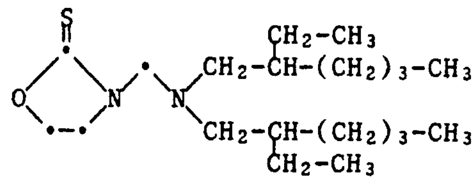 oder

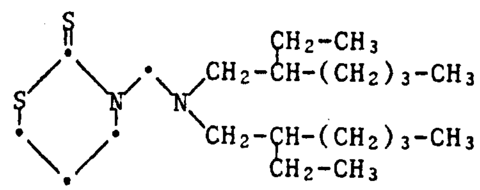 .

Eine weitere Ausführungsform stellt eine Zusammensetzung dar, worin als Additiv mindestens eine Verbindung der Formeln I oder II, bevorzugt jedoch mindestens eine Verbindung der Formel I, enthalten ist.

Eine speziell bevorzugte Ausführungsform stellt eine Zusammensetzung dar, worin in den Verbindungen der Formeln I und II n Null, $R^1$ und $R^3$ Wasserstoff, $R^4$ und $R^5$ unabhängig voneinander $C_1$-$C_{18}$-Alkyl oder $C_3$-$C_{18}$-Alkenyl und $R^6$ $C_3$-$C_{18}$-Alkenyl und X Schwefel bedeuten.

Beispiele für Verbindungen der Formeln I, II oder III sind:

3-Dibutylaminomethyl-thiazolidin-2-thion
3-[(Di-2-ethylhexyl)aminomethyl]-thiazolidin-2-thion
3-Tridecylaminomethyl-thiazolidin-2-thion
3-Oleylaminomethyl-thiazolidin-2-thion
3-[N-Phenyl-N-methyl-aminomethyl]-5-[tert.-butyl-thio-methyl]-thiazolidin-2-thion
3-Piperidinomethyl-5-acetoxymethyl-oxazolidin-2-thion
3-Morpholinomethyl-5,6-tetramethylen-perhydrooxazin-2-thion
3-Di-tert.-dodecylmethylamino-4-methyl-5-butyl-thiazolidin-2-thion
3-Dibutylaminomethyl-5-vinyl-oxazolidin-2-thion
3-(4-Oxa-octyl)aminomethyl-thiazolidin-2-thion
Bis-[(2-thiono-oxazolidino)methyl]-benzylamin
Bis-[(2-thiono-thiazolidino)methyl]-oleylamin
Bis-[(2-thiono-5-isopropyl-oxazolidino)methyl]-anilin
Bis-[(2-thiono-5-methyl-thiazolidino)methyl]-butylamin
Ethylen-N,N′-[bis(2-thiono-5,6-dimethyl-perhydrooxazino)-methyl]-N,N′-dibutyl-diamin
4,4′-Biphenylylen-N,N′-[bis-(2-thiono-thiazolidino)methyl]-diamin
1,4-Bis[(2-thiono-5-(4′nonylphenoxymethyl)-thiazolidino)methyl]-piperazin

Die Verbindungen der Formeln I, II und III sind z.T. bekannt und z.T. neu. Bekannt sind 3-Dimethylaminomethyl-5-phenyl-oxazolidin-2-thion, 3-Cyclohexylaminomethyl-5-methyl-oxazolidin-2-thion, 3-Piperidinomethyl-5-methyl-oxazolidin-2-thion, 3-Piperidinomethyl-oxazolidin-2-thion, 3-Morpholinomethyl-5-methyl-oxazolidin-2-thion, 3-Morpholinomethyl-4-methyl-oxazolidin-2-thion, 3-Morpholinomethyl-oxazolidin-2-thion, 3-[(4′-Methylpiperazino)methyl]-5-methyl-oxazolidin-2-thion, 3-Dimethylaminomethyl-thiazolidin-2-thion, 3-Diethylaminomethyl-thiazolidin-2-thion, 3-Piperidinomethyl-thiazolidin-2-thion, 3-Morpholinomethyl-5-methyl-thiazolidin-2-thion, 3-Morpholinomethyl-4-methyl-thiazolidin-2-thion, 3-Morpholinomethyl-5-phenyl-thiazolidin-2-thion, 3-[(4′-Methylpiperazino)methyl]-thiazolidin-2-thion, Bis-[(2-thiono-5-methyl-oxazolidino)-methyl]-methylamin, Bis-[(2-thiono-5-methyl-oxazolidino)-methyl]-isopropylamin und Bis[(2-thiono-thiazolidino)methyl]-methylamin.

Daher betrifft die vorliegende Erfindung im weiteren auch Verbindungen der Formeln I*, II* und III*,

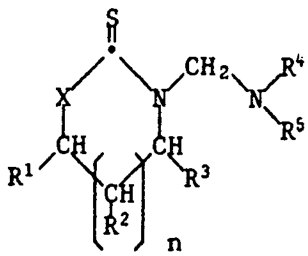

I*

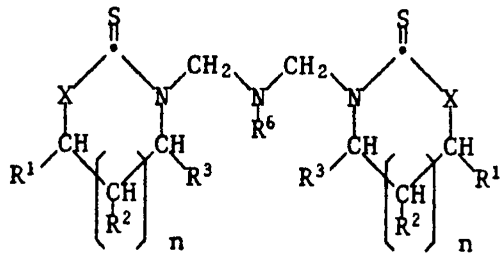

II*

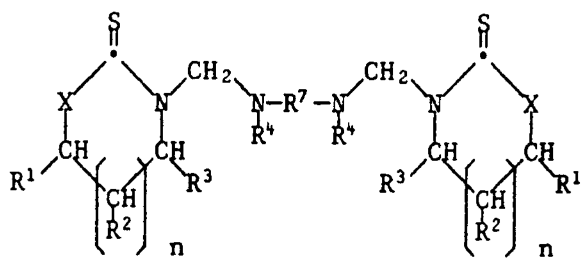

III*

worin $R^1$, $R^2$, $R^3$, $R^4$, $R^5$ und $R^6$ unabhängig voneinander $C_1$-$C_{24}$-Alkyl oder $C_1$-$C_{24}$-Alkyl das durch Oxo- oder Thionogruppen substituiert ist oder $C_3$-$C_{24}$-Alkyl, das durch

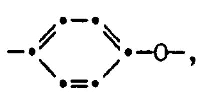
,

-O-, -S- und/oder -N($R^8$)-, mit $R^8$ als Wasserstoff oder $C_1$-$C_{12}$-Alkyl, unterbrochen ist oder $C_3$-$C_{24}$-Alkyl, das durch

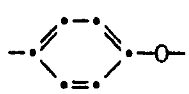
,

-O-, -S- und/oder -N($R^8$)-, mit $R^8$ als Wasserstoff oder $C_1$-$C_{12}$-Alkyl, unterbrochen ist und das durch Oxo- oder Thionogruppen substituiert ist, oder $C_2$-$C_{24}$-Alkenyl, oder unsubstituiertes oder durch ein oder zwei $C_1$-$C_{12}$-Alkyl-, $C_1$-$C_4$-Alkoxy-, $C_2$-$C_{24}$-Carboalkoxy- oder Nitrogruppen substituiertes Phenyl oder Naphthyl, oder $C_7$-$C_{10}$-Phenylalkyl bedeuten und $R^1$, $R^2$, $R^3$ und $R^4$ zusätzlich Wasserstoff bedeutet, oder $R^1$ und $R^2$ bzw. $R^1$ und $R^3$ im Fall von n gleich Null zusammen mit den C-Atomen, an die sie gebunden sind, einen 5- oder 6-gliedrigen aliphatischen Ring bilden oder einen 5- oder 6-gliedrigen aliphatischen Ring, der durch Oxo- oder Thionogruppen substituiert ist, bilden oder einen 5- oder 6-gliedrigen aliphatischen Ring bilden, der durch -N($R^8$)-, mit $R^8$ in der vorstehenden Bedeutung, -O-oder -S- unterbrochen ist oder einen 5- oder 6-gliedrigen aliphatischen Ring bilden, der durch -N($R^8$)-, mit $R^8$ in der vorstehenden Bedeutung, -O- oder -S-unterbrochen und durch Oxo-oder Thionogruppen substituiert ist, oder worin $R^4$ und $R^5$ zusammen mit dem N-Atom, an das sie gebunden sind, einen 5- bis 7-gliedrigen aliphatischen-heterocyclischen Ring bilden, oder worin $R^4$ und $R^5$ zusammen mit dem N-Atom, an das sie gebunden sind, einen 5- bis 7-

gliedrigen aliphatischen-heterocyclischen Ring bilden und der Ring durch Oxo- oder Thionogruppen substituiert ist und/oder zusätzlich zu dem N-Atom noch weitere Heteroatome [$N(R^8)$, O, S] enthält, und worin $R^7$ $C_2$-$C_{12}$-Alkylen, $C_2$-$C_{12}$-Alkylen, das durch -$N(R^8)$-, -O-, und/oder -S-unterbrochen ist, $C_2$-$C_{12}$-Alkylen, das Oxo- oder Thionogruppen enthält, $C_6$-$C_{15}$-Cycloalkylen, $C_6$-$C_{15}$-Arylen, Carbonyl oder Thiocarbonyl bedeutet oder die Gruppe -$N(R^4)$-$R^7$- $N(R^4)$- einen Piperazin-1,4-diylrest darstellt, der durch eine oder mehrere Methylgruppen substituiert sein kann, ferner X Sauerstoff oder Schwefel und n die Zahl Null oder 1 bedeuten, wobei für den Fall, dass $R^1$ und $R^3$ Wasserstoff, $C_1$-$C_{24}$-Alkyl, $C_1$-$C_{24}$-Alkyl, das durch Oxo- oder Thionogruppen substituiert ist, unsubstituiertes oder durch ein oder zwei $C_1$-$C_{12}$-Alkyl-, $C_1$-$C_4$-Alkoxy-, $C_2$-$C_{24}$-Carbalkoxy- oder Nitrogruppen substituiertes Phenyl oder Naphthyl oder $C_7$-$C_{10}$-Phenylalkyl darstellen, $R^4$ ungleich Wasserstoff ist und $R^4$ und $R^5$ nicht $C_1$-$C_{24}$-Alkyl, $C_1$-$C_{24}$-Alkyl, das durch Oxo- oder Thionogruppen substituiert ist, Phenyl, Naphthyl oder $C_7$-$C_{10}$-Phenylalkyl bedeuten oder $R^4$ und $R^5$ zusammen mit dem N-Atom, an das sie gebunden sind, keinen Pyrrolidin-, Piperidin-, Morpholin- oder N-substituierten Piperazinring bilden, und mit der Massgabe, dass die Verbindungen nicht Bis-[(2-thiono-5-methyl-oxazolidino)methyl]-methylamin, Bis-[(2-thiono-5-methyl-oxazolidino)methyl]isopropylamin oder Bis-[-(2-thiono-thiazolidino)methyl]-methylamin bedeuten.

Bezüglich der Bevorzugungen für bestimmte Verbindungen der Formeln I*, II* und III* gelten diejenigen, wie sie für die Komponenten der Formeln I, II und III in den Schmiermittel- oder Hydraulikölzusammensetzungen definiert sind, in analoger Weise.

Ganz besonders bevorzugte Verbindungen sind

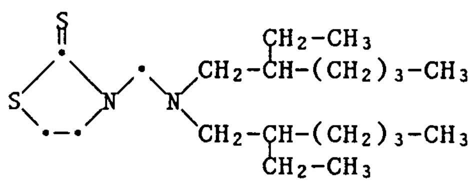

,

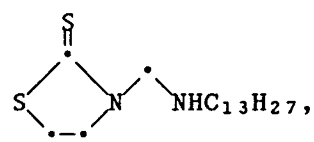

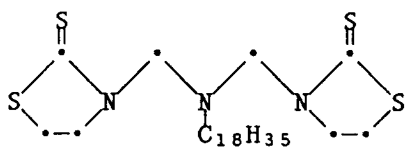

,

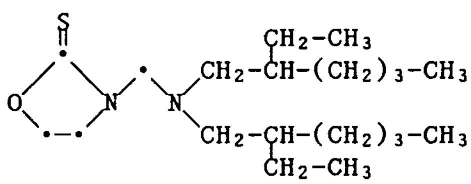

und

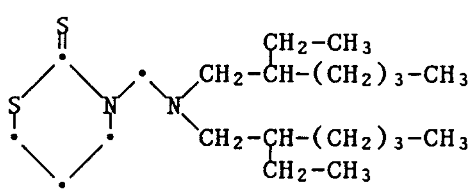

.

Die Herstellung der Verbindungen der Formel I, II oder III erfolgt nach an sich bekannter Weise.

So können beispielsweise die heterocyclischen Ringe wie Oxazolidin-2-thion oder Perhydrooxazin-2-thion nach der in Fülöp et al. in Synthesis (1985) 1149-51 beschriebenen Methode hergestellt werden.

Thiazolidin-2-thion und Derivate davon können z.B. nach dem in der DE-A 2,701,215 beschriebenen Verfahren hergestellt werden.

Perhydrothiazin-2-thion und dessen Derivate können beispielsweise nach der in der US-A 2,920,996 beschriebenen Methode hergestellt werden.

Die erfindungsgemässen Aminomethylderivate werden durch eine Mannich-Reaktion der vorstehend erwähnten Heterocyclen mit Formaldehyd und einem primären oder sekundären Amin in üblicher Weise hergestellt.

Die Verbindungen der Formel II werden analogerweise mit Formaldehyd und einem primären Amin hergestellt, wobei das Molverhältnis Heterocyclus: Formaldehyd: Amin 2:2:1 beträgt.

Die Verbindungen der Formel III werden durch eine Mannich-Reaktion der Heterocyclen mit Formaldehyd und einem primären oder sekundären Diamin hergestellt, wobei das Molverhältnis Heterocyclus: Formaldehyd: Amin 2:2:1 beträgt.

Die Verbindungen der Formeln I, II oder III sind als Verschleissschutz-und Hochdruckadditive für Schmiermittel und Hydrauliköle, vorzugsweise für Schmiermittel, hervorragend geeignet.

Ein weiterer Gegenstand der vorliegenden Erfindung ist deshalb die Verwendung von mindestens einer Verbindung der Formeln I, II oder III als Hochdruck- oder verschleissmindernde Zusätze zu mineralischen oder synthetischen Schmiermitteln oder Hydraulikölen.

Die Verbindungen der Formeln, I, II oder III sind in Schmiermitteln und Hydraulikölen in ausreichender Menge löslich und werden beispielsweise in einer Konzentration von 0,05 bis 5 Gew.-%, bevorzugt in einer Konzentration von 0,1 bis 3 Gew.-%, bezogen auf das Gesamtgewicht der Schmiermittel- bzw. Hydraulikölzusammensetzung, eingesetzt.

Die in Frage kommenden Schmiermitteln bzw. Hydrauliköle sind dem Fachmann geläufig und z.B. im "Schmiermittel Taschenbuch" (Hüthig Verlag, Heidelberg, 1974) resp. in "Ullmanns Encyclopädie der technischen Chemie" Bd. 13, Seiten 85-94 (Verlag Chemie, Weinheim, 1977) beschrieben.

Besonders geeignet sind neben Mineralölen z.B. Poly-α-olefine, Schmiermittel auf Esterbasis, Phosphatester, Glykole, Polyglykole und Polyalkylenglykole.

Die Schmiermittel können zusätzlich andere Additive enthalten, die zugegeben werden, um die Grundeigenschaften von Schmierstoffen und Hydraulikölen noch weiter zu verbessern; dazu gehören Antioxidantien, Metallpassivatoren, Rostinhibitoren, Viskositätsindex-Verbesserer, Stockpunkterniedriger, Dispergiermittel, Detergentien, weitere Hochdruck-Zusätze sowie andere Verschleissschutz-Additive.

Beispiele für phenolische Antioxidantien

1. Alkylierte Monophenole

2,6-Di-tert-butyl-4-methylphenol
2,6-Di-tert-butylphenol
2-tert-Butyl-4,6-dimethylphenol
2,6-Di-tert-butyl-4-ethylphenol
2,6-Di-tert-butyl-4-isopropylphenol
2,6-Di-tert-butyl-4-n-butylphenol
2,6-Di-tert-butyl-4-iso-n-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert-butyl-4-methoxymethylphenol
o-tert-Butylphenol

2. Alkylierte Hydrochinone

2,6-Di-tert-butyl-4-methoxyphenol
2,5-Di-tert-butyl-hydrochinon
2,5-Di-tert-amyl-hydrochinon
2,6-Diphenyl-4-octadecyloxyphenol

3. Hydroxylierte Thiodiphenylether

2,2′-Thio-bis-(6-tert-butyl-4-methylphenol)
2,2′-Thio-bis-(4-octylphenol)
4,4′-Thio-bis-(6-tert-butyl-3-methylphenol)
4,4′-Thio-bis-(6-tert-butyl-2-methylphenol)

4. Alkyliden-Bisphenole

2,2′-Methylen-bis-(6-tert-butyl-4-methylphenol)
2,2′-Methylen-bis-(6-tert-butyl-4-ethylphenol)
2,2′-Methylen-bis-[4 methyl-6-(α-methylcyclohexyl)-phenol]
2,2′-Methylen-bis-(4-methyl-6-cyclohexylphenol)
2,2′-Methylen-bis-(6-nonyl-4-methylphenol)

2,2′-Methylen-bis-(4,6-di-tert-butylphenol)
2,2′-Ethyliden-bis-(4,6-di-tert-butylphenol)
2,2′-Ethyliden-bis-(6-tert-butyl-4-iso-butylphenol)
2,2′-Methylen-bis-[6-(α-methylbenzyl)-4-nonylphenol]
2,2′-Methylen-bis-[6-(α,α-dimethylbenzyl)-4-nonylphenol]
4,4′-Methylen-bis-(2,6-di-tert-butylphenol)
4,4′-Methylen-bis-(6-tert-butyl-2-methylphenol)
1,1-Bis-(5-tert-butyl-4-hydroxy-2-methylphenyl)-butan
2,6-Di-(3-tert-butyl-5-methyl-2-hydroxybenzyl)-4-methylphenol
1,1,3-Tris-(5-tert-butyl-4-hydroxy-2-methylphenyl)-3-n-dodecylmercaptobutan Ethylenglycol-bis-[3,3-bis-(3′-tert-butyl-4′-hydroxyphenyl)-butyrat]
Di-(3-tert-butyl-4-hydroxy-5-methylphenyl)-dicyclopentadien
Di-[2-(3′-tert-butyl-2′-hydroxy-5′-methyl-benzyl)-6-tert-butyl-4-methylphenyl]-terephthalat.

5. Benzylverbindungen

1,3,5-Tri(3,5-di-tert-butyl-4-hydroxybenzyl)-2,4,6-trimethylbenzol
Di-(3,5-di-tert-butyl-4-hydroxybenzyl)-sulfid
3,5-Di-tert-butyl-4-hydroxybenzyl-mercaptoessigsäure-isooctylester
Bis-(4-tert-butyl-3-hydroxy-2,6-dimethylbenzyl)-dithiol-terephthalat
1,3,5-Tris-(3,5-di-tert-butyl-4-hydroxybenzyl)-isocyanurat
1,3,5-Tris-(4-tert-butyl-3-hydroxy-2,6-dimethylbenzyl)-isocyanurat
3,5-Di-tert-butyl-4-hydroxybenzyl-phosphonsäure-dioctadecylester
3,5-Di-tert-butyl-4-hydroxybenzyl-phosphonsäure-monoethylester Calcium-salz.

6. Acylaminophenole

4-Hydroxy-laurinsäureanilid
4-Hydroxy-stearinsäureanilid
2,4-Bis-octylmercapto-6-(3,5-di-tert-butyl-4-hydroxyanilino)-striazin
N-(3,5-di-tert-butyl-4-hydroxyphenyl)-carbaminsäureoctylester.

7. Ester der β-(3,5-Di-tert-butyl-4-hydroxyphenyl)-propionsäure

mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

8. Ester der β-(5-tert-butyl-4-hydroxy-3-methylphenyl)-propion säure

mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

9. Amide der β-(3,5-Di-tert-butyl-4-hydroxyphenyl)-propionsäure,

wie z.B.
N,N′-Di-(3,5-di-tert-butyl-4-hydroxyphenylpropionyl)-hexamethylendiamin
N,N′-Di-(3,5-di-tert-butyl-4-hydroxyphenylpropionyl)-trimethylendiamin
N,N′-Di-(3,5-di-tert-butyl-4-hydroxyphenylpropionyl)-hydrazin.

Beispiele für aminische Antioxidantien:

N,N′-Di-isopropyl-p-phenylendiamin
N,N′-Di-sec-butyl-p-phenylendiamin
N,N′-Bis(1,4-dimethyl-pentyl)-p-phenylendiamin
N,N′-Bis(1-ethyl-3-methyl-pentyl)-p-phenylendiamin
N,N′-Bis(1-methyl-heptyl)-p-phenylendiamin
N,N′-Diphenyl-p-phenylendiamin
N,N′-Di-(naphthyl-2)-p-phenylendiamin
N-Isopropyl-N′-phenyl-p-phenylendiamin
N-(1,3-Dimethyl-butyl)-N′-phenyl-p-phenylendiamin
N-(1-Methyl-heptyl)-N′-phenyl-p-phenylendiamin
N-Cyclohexyl-N′-phenyl-p-phenylendiamin
4-(p-Toluol-sulfonamido)-diphenylamin
N,N′-Dimethyl-N,N′-di-sec-butyl-p-phenylendiamin
Diphenylamin
4-Isopropoxy-diphenylamin
N-Phenyl-1-naphthylamin
N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert-butyl-4-dimethylamino-methyl-phenol
2,4′-Diamino-diphenylmethan
4,4′-Diamino-diphenylmethan
N,N,N′,N′-Tetramethyl-4,4′-diamino-diphenylmethan
1,2-Di-[(2-methyl-phenyl)-amino]-ethan
1,2-Di-(phenylamino)-propan
(o-Tolyl)-biguanid
Di-[4-(1′,3′-dimethyl-butyl)-phenyl)amin
tert-octyliertes N-Phenyl-1-naphthylamino
Gemisch aus mono- und dialkylierten tert-Butyl-/tert-Octyldiphenylaminen.

Beispiele für Metallpassivatoren sind:

für Kupfer, z.B.:
Triazol, Benztriazol und deren Derivate, 2-Mercaptobenzthiazol, 2,5-Dimercaptothiadiazol, Salicyliden-propylendiamin, Salze von Salicylaminoguanidin.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Dodecenylbernsteinsäure-anhydrid, Alkenylbernsteinsäure-Halbester, 4-Nonylphenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.:
Substituierte Imidazoline und Oxazoline.

c) Phosphorhaltige Verbindungen, z.B.:
Aminsalze von Phosphorsäurepartialestern.
d) Schwefelhaltige Verbindungen, z.B.:
Barium-dinonylnaphthalin-sulfonate, Calciumpetroleum-sulfonate.

Beispiele für Viskositätsindex-Verbesserer sind z.B.

Polymethacrylate, Vinylpyrrolidon/Methacrylat-Copolymere, Polybutene, Olefin-Copolymere, Styrol/Acrylat-Copolymere.

Beispiele für Stockpunkterniedriger sind z.B.:

Polymethacrylat, alkylierte Naphthalinderivate.

Beispiele für Dispergiermittel/Tenside sind z.B.:

Polybutenylbernsteinsäure-imide, Polybutenylphosphonsäurederivate, basische Magnesium-, Calcium-, und Bariumsulfonate und -phenolate.

Beispiele für Verschleissschutz-Additive sind z.B.:

Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldisulfide.

In den nachfolgenden Beispielen beziehen sich Teile und Prozente auf das Gewicht, sofern nichts anderes angegeben ist.

Beispiel 1

3-(Dibutylaminomethyl)-thiazolin-2-thion. Eine Suspension von 47,3 g (0,4 Mol) 2-Mercaptothiazolin mit 52,3 g (0,4 Mol) Dibutylamin und 34,3 g (0,41 Mol) 36 % wässrigem Formaldehyd in 150 ml Toluol wird 5 Stunden bei 50 °C gerührt. Dann wird das Wasser von der klaren Toluolphase abgetrennt und diese wird bei reduziertem Druck eingedampft. Es werden so 94,8 g (Ausbeute 91 %) 3-(Dibutylaminomethyl)-thiazolin-2-thion als gelbliches, dünnflüssiges Oel erhalten. $n_D^{20}$ 1,5512.

In analoger Weise werden die Beispiele 2-7 hergestellt. Die Resultate sind in Tabelle 1 wiedergegeben.

Tabelle 1

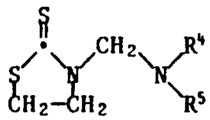

| Beispiel | 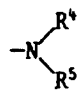 | Ausbeute [%] | Aussehen | $n_D^{20}$ | Analyse | C | H | N | S |
|---|---|---|---|---|---|---|---|---|---|
| | | | | | berechnet<br>gefunden | | | | |
| 1 | 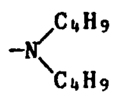 | 91 | blass-gelbes Oel | 1,5512 | | 55,34<br>55,41 | 9,29<br>9,27 | 10,76<br>10,90 | 24,62<br>24,62 |
| 2 | 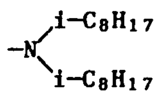 | 98 | blass-gelbes Oel | 1,5280 | | 64,46<br>64,65 | 10,82<br>10,63 | 7,52<br>7,60 | 17,21<br>16,62 |
| 3 | 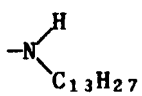 | 93 | hell-gelbes Oel | 1,5440 | | 61,76<br>60,89 | 10,37<br>10,24 | 8,47<br>8,76 | 19,40<br>20,10 |
| 4 | 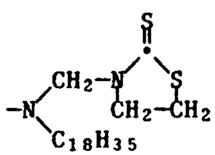 | | hell-gelbes, hoch-viskoses Oel | 1,5718 | | | | | |

Tabelle 1 (Fortsetzung)

| Beispiel | 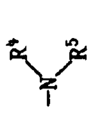 | Ausbeute [%] | Aussehen | $n_D^{20}$ | | Analyse C | H | N | S |
|---|---|---|---|---|---|---|---|---|---|
| | | | | | berechnet<br>gefunden | | | | |
| 5 | 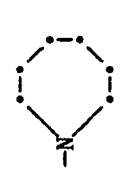 | 98 | gelbes Oel | 1,6103 | | 52,13<br>52,26 | 7,88<br>7,94 | 12,16<br>12,14 | 27,83<br>27,72 |
| 6 | 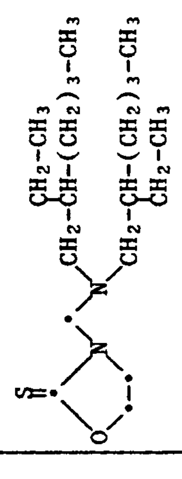 | 99 | blass-gelbes Oel | 1,4988 | | 67,36<br>67,73 | 11,31<br>11,27 | 7,86<br>8,18 | 8,99<br>8,84 |
| 7 | 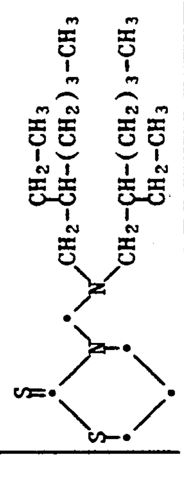 | 98 | blass-gelbes Oel | 1,5309 | | 65,22<br>65,35 | 10,95<br>10,81 | 7,24<br>7,28 | 16,58<br>16,48 |

Beispiel 8

Mit dem Shell-Vierkugel Apparat (IP 239/73 Extreme pressure and wear lubricant test for oils and greases - four ball machine; ASTM-D 2783-81) werden folgende Werte bestimmt:

1. W.L. = Weld load (Schweisslast in (kg)). Das ist die Last, bei der die 4 Kugeln innerhalb von 10 Sekunden zusammenschweissen.
2. W.S.D. = Wear Sear Diameter in (mm). Das ist der mittlere Verschleissdurchmesser bei einer Belastung von 40 kg während 1 h.

Als Basisöl wird Catenex® P 941 der Firma Shell verwendet.
Die Resultate sind in Tabelle 2 wiedergegeben.

Tabelle 2

| Additiv aus Beispiel | Konzentration [%] | W.L. [kg] | W.S.D. [mm] |
|---|---|---|---|
| ohne | — | 160 | 0,9 |
| 1 | 1,0<br>2,5 | 220<br>260 | 0,60 |
| 2 | 1,0<br>2,5 | 220<br>260 | 0,7 |
| 3 | 1,0<br>2,5 | 240<br>260 | 0,7 |
| 4 | 1,0<br>2,5 | 220<br>260 | 0,75 |

Tabelle 2 (Fortsetzung)

| Additiv aus Beispiel | Konzentration [%] | W.L. [kg] | W.S.D. [mm] |
|---|---|---|---|
| 5 | 1,0 | 220 | 0,70 |
| 6 | 1,0 | 200 | 0,57 |
| 7 | 1,0 | 200 | 0,75 |

**Patentansprüche**
**Patentansprüche für folgende Vertragsstaaten : BE, DE, FR, GB, IT, NL**

**1.** Zusammensetzung enthaltend ein oder mehrere Schmiermittel oder Hydrauliköle auf der Basis von

Mineralöl, synthetischen Oelen oder Gemischen davon und mindestens eine Verbindung der Formeln I, II und/oder III,

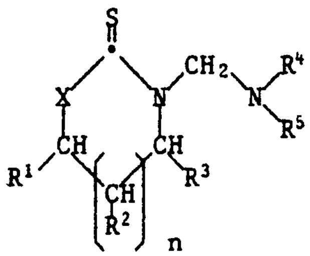

I

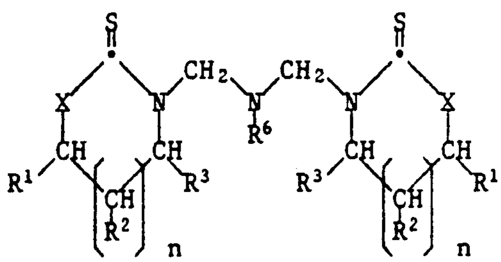

II

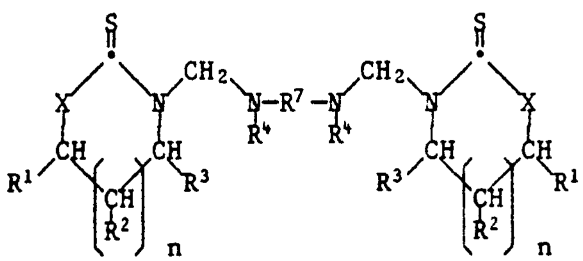

III

worin
$R^1$, $R^2$, $R^3$, $R^4$, $R^5$ und $R^6$ unabhängig voneinander $C_1$-$C_{24}$-Alkyl oder $C_1$-$C_{24}$-Alkyl das durch Oxo- oder Thionogruppen substituiert ist oder $C_3$-$C_{24}$-Alkyl, das durch

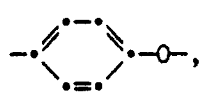

-O-, -S- und/oder -N($R^8$)-, mit $R^8$ als Wasserstoff oder $C_1$-$C_{12}$-Alkyl, unterbrochen ist oder $C_3$-$C_{24}$-Alkyl, das durch

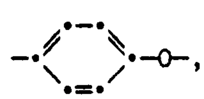

-O-, -S- und/oder -N($R^8$)-, mit $R^8$ als Wasserstoff oder $C_1$-$C_{12}$-Alkyl, unterbrochen ist und das durch Oxo-oder Thionogruppen substituiert ist, oder $C_2$-$C_{24}$-Alkenyl, oder unsubstituiertes oder durch ein oder zwei $C_1$-$C_{12}$-Alkyl-, $C_1$-$C_4$-Alkoxy-, $C_2$-$C_{24}$-Carboalkoxy- oder Nitrogruppen substituiertes Phenyl oder Naphthyl, oder $C_7$-$C_{10}$-Phenylalkyl bedeuten und $R^1$, $R^2$, $R^3$ und $R^4$ zusätzlich Wasserstoff bedeutet, oder $R^1$ und $R^2$ bzw. $R^1$ und $R^3$ im Fall von n gleich Null, zusammen mit den C-Atomen, an die sie gebunden sind, einen 5- oder 6-gliedrigen aliphatischen Ring bilden oder einen 5- oder 6-gliedrigen aliphatischen Ring, der durch Oxo- oder Thionogruppen substituiert ist, bilden oder einen 5- oder 6-gliedrigen aliphatischen Ring bilden, der durch -N($R^8$)-, mit $R^8$ in der vorstehenden Bedeutung, -O-oder -S- unterbrochen ist oder einen 5- oder 6-gliedrigen aliphatischen Ring bilden, der durch -N($R^8$)-, mit $R^8$ in der vorstehenden Bedeutung, -O-oder -S- unterbrochen und durch Oxo- oder Thionogruppen substituiert ist, oder worin $R^4$ und $R^5$ zusammen mit den N-Atom, an das sie gebunden sind, einen 5- bis 7-gliedrigen aliphatischen-heterocyclischen Ring bilden, oder worin $R^4$ und $R^5$ zusammen mit dem

N-Atom, an das sie gebunden sind, einen 5- bis 7-gliedrigen aliphatischen-heterocyclischen Ring bilden und der Ring durch Oxo- oder Thionogruppen substituiert ist und/oder zusätzlich zu dem N-Atom noch weitere Heteroatome [N($R^8$), O, S] enthält, und worin $R^7$ $C_2$-$C_{12}$-Alkylen, $C_2$-$C_{12}$-Alkylen, das durch -N($R^8$)-, -O-, und/oder -S-unterbrochen ist, $C_2$-$C_{12}$-Alkylen, das Oxo- oder Thionogruppen enthält oder $C_2$-$C_{12}$-Alkylen, das durch -N($R^8$)-, -O- und/oder -S- unterbrochen ist und das Oxo- oder Thionogruppen enthält, $C_6$-$C_{15}$-Cycloalkylen, $C_6$-$C_{15}$-Arylen, Carbonyl oder Thiocarbonyl bedeutet oder die Gruppe -N($R^4$)-$R^7$-N($R^4$)-einen Piperazin-1,4-diylrest oder einen Piperazin-1,4-diylrest, der durch eine oder mehrere Methylgruppen substituiert ist, darstellt, und X Sauerstoff oder Schwefel und n die Zahlen Null oder 1 bedeuten.

2. Zusammensetzung gemäss Anspruch 1, worin in den Verbindungen der Formeln I, II oder III n gleich 1 ist.

3. Zusammensetzung gemäss Anspruch 2, worin in den Verbindungen der Formeln I, II oder III $R^2$ Wasserstoff, $C_1$-$C_{12}$-Alkyl, Phenyl oder Benzyl bedeutet.

4. Zusammensetzung gemäss Anspruch 2, worin in den Verbindungen der Formeln I, II oder III $R^1$ und $R^2$ zusammen mit den C-Atomen, an die sie gebunden sind, einen 6-gliedrigen aliphatischen Ring bilden.

5. Zusammensetzung gemäss Anspruch 1, worin in den Verbindungen der Formeln I, II oder III n gleich Null ist.

6. Zusammensetzung gemäss Anspruch 1, worin in den Verbindungen der Formeln I, II oder III $R^1$ Wasserstoff, $C_1$-$C_{12}$-Alkyl, $C_1$-$C_{12}$-Alkyl das durch eine Oxogruppe substituiert ist, $C_3$-$C_{12}$-Alkyl, das durch

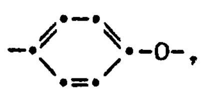

-O- oder -S- unterbrochen ist oder $C_3$-$C_{12}$-Alkyl, das durch

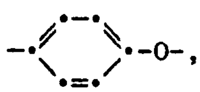

-O- oder -S- unterbrochen und durch eine Oxogruppe substituiert ist, Phenyl oder Benzyl bedeutet.

7. Zusammensetzung gemäss Anspruch 1, worin in den Verbindungen der Formeln I, II oder III $R^3$ Wasserstoff oder $C_1$-$C_4$-Alkyl bedeutet.

8. Zusammensetzung gemäss Anspruch 1, worin in den Verbindungen der Formeln I oder III $R^4$ Wasserstoff, $C_1$-$C_{12}$-Alkyl, $C_3$-$C_{18}$-Alkenyl, Phenyl oder Benzyl bedeutet.

9. Zusammensetzung gemäss Anspruch 1, worin in der Verbindung der Formel I $R^5$ $C_1$-$C_{18}$-Alkyl, $C_3$-$C_{18}$-Alkenyl, Phenyl oder Benzyl bedeutet.

10. Zusammensetzung gemäss Anspruch 1, worin in der Verbindung der Formel I $R^4$ und $R^5$ zusammen mit dem N-Atom, an das sie gebunden sind einen Piperidin-, Perhydroazepin-, Morpholin-, Piperazin- oder einen 4-N-Methylpiperazinring bilden.

11. Zusammensetzung gemäss Anspruch 1, worin in der Verbindung der Formel II $R^6$ $C_1$-$C_{12}$-Alkyl, $C_3$-$C_{18}$-Alkenyl, Phenyl oder Benzyl bedeutet.

12. Zusammensetzung gemäss Anspruch 1, worin in den Verbindungen der Formeln I, II oder III X Schwefel bedeutet.

**13.** Zusammensetzung gemäss Anspruch 1, worin die Menge der Verbindungen der Formeln I, II oder III 0,05 bis 5 Gew.-%, bezogen auf das Gesamtgewicht der Zusammensetzung, beträgt.

**14.** Zusammensetzung gemäss Anspruch 1, worin in den Verbindungen der Formeln I und II n Null, $R^1$ und $R^3$ Wasserstoff, $R^4$ und $R^5$ unabhängig voneinander $C_1$-$C_{18}$-Alkyl oder $C_3$-$C_{18}$-Alkenyl und $R^6$ $C_3$-$C_{18}$-Alkenyl und X Schwefel bedeuten.

**15.** Verwendung von mindestens einer Verbindung der Formeln I, II oder III nach Anspruch 1 als Hochdruck- oder verschleissmindernde Zusätze zu mineralischen oder synthetischen Schmiermitteln oder Hydraulikölen.

**16.** Verbindungen der Formeln $I^*$, $II^*$ oder $III^*$,

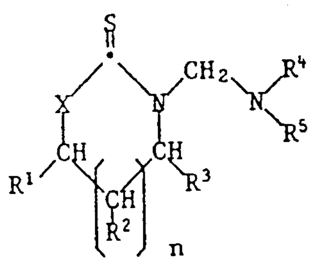

$I^*$

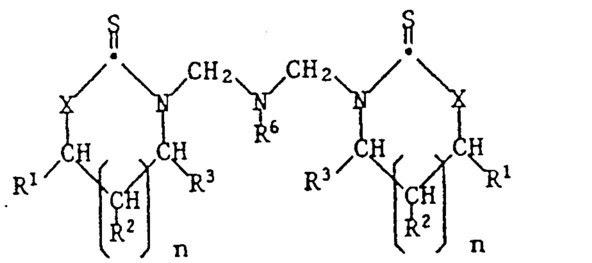

$II^*$

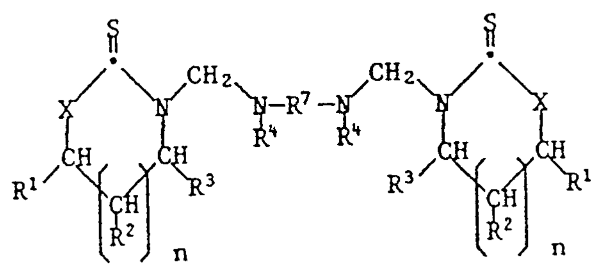

$III^*$

worin
$R^1$, $R^2$, $R^3$, $R^4$, $R^5$ und $R^6$ unabhängig voneinander $C_1$-$C_{24}$-Alkyl oder $C_1$-$C_{24}$-Alkyl das durch Oxo- oder Thionogruppen substituiert ist oder $C_3$-$C_{24}$-Alkyl, das durch

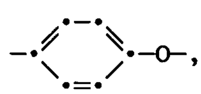

-O-, -S- und/oder oder -N($R^8$)-, mit $R^8$ als Wasserstoff oder $C_1$-$C_{12}$-Alkyl, unterbrochen ist oder $C_3$-$C_2$-Alkyl, das durch

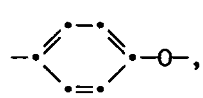

-O-, -S- und/oder -N($R^8$)-, mit $R^8$ als Wasserstoff oder $C_1$-$C_{12}$-Alkyl, unterbrochen ist und das durch Oxo-oder Thionogruppen substituiert ist, oder $C_2$-$C_{24}$-Alkenyl, oder unsubstituiertes oder durch ein oder

zwei $C_1$-$C_{12}$-Alkyl-, $C_1$-$C_4$-Alkoxy-, $C_2$-$C_{24}$-Carboalkoxy- oder Nitrogruppen substituiertes Phenyl oder Naphtyl, oder $C_7$-$C_{10}$-Phenylalkyl bedeuten und $R^1$, $R^2$, $R^3$ und $R^4$ zusätzlich Wasserstoff bedeutet, oder $R^1$ und $R^2$ bzw. $R^1$ und $R^3$ im Fall von n gleich Null, zusammen mit den C-Atomen, an die sie gebunden sind, einen 5- oder 6-gliedrigen aliphatischen Ring bilden oder einen 5- oder 6-gliedrigen aliphatischen Ring, der durch Oxo- oder Thionogruppen substituiert ist, bilden oder einen 5- oder 6-gliedrigen aliphatischen Ring bilden, der durch -N($R^8$)-, mit $R^8$ in der vorstehenden Bedeutung, -O-oder -S- unterbrochen ist oder einen 5- oder 6-gliedrigen aliphatischen Ring bilden, der durch -N($R^8$)-, mit $R^8$ in der vorstehenden Bedeutung, -O-oder -S-unterbrochen und durch Oxo-oder Thionogruppen substituiert ist, oder worin $R^4$ und $R^5$ zusammen mit dem N-Atom, an das sie gebunden sind, einen 5- bis 7-gliedrigen aliphatischen-heterocyclischen Ring bilden, oder worin $R^4$ und $R^5$ zusammen mit dem N-Atom, an das sie gebunden sind, einen 5- bis 7-gliedrigen aliphatischen-heterocyclischen Ring bilden und der Ring durch Oxo- oder Thionogruppen substituiert ist und/oder zusätzlich zu dem N-Atom noch weitere Heteroatome [N($R^8$), O, S] enthält, und worin $R^7$ $C_2$-$C_{12}$-Alkylen, $C_2$-$C_{12}$-Alkylen, das durch -N($R^8$)-, -O-, und/oder -S-unterbrochen ist, $C_2$-$C_{12}$-Alkylen, das Oxo- oder Thionogruppen enthält oder $C_2$-$C_{12}$-Alkylen, das durch -N($R^8$)-, -O- und/oder -S-unterbrochen ist und das Oxo- oder Thionogruppen enthält, $C_6$-$C_{15}$-Cycloalkylen, $C_6$-$C_{15}$-Arylen, Carbonyl oder Thiocarbonyl bedeutet oder die Gruppe -N($R^4$)-$R^7$-N($R^4$)- einen Piperazin-1,4-diylrest oder einen Piperazin-1,4-diylrest, der durch eine oder mehrere Methylgruppen substituiert ist, darstellt, und X Sauerstoff oder Schwefel und n die Zahlen Null oder 1 bedeuten, wobei für den Fall, dass $R^1$ und $R^3$ Wasserstoff, $C_1$-$C_{24}$-Alkyl, $C_1$-$C_{24}$-Alkyl, das durch Oxo- oder Thionogruppen substituiert ist, unsubstituiertes oder durch ein oder zwei $C_1$-$C_{12}$-Alkyl-, $C_1$-$C_4$-Alkoxy-, $C_2$-$C_2$-Carbalkoxy- oder Nitrogruppen substituiertes Phenyl oder Naphthyl oder $C_7$-$C_{10}$-Phenylalkyl darstellen, $R^4$ ungleich Wasserstoff ist und $R^4$ und $R^5$ nicht $C_1$-$C_{24}$-Alkyl, $C_1$-$C_2$-Alkyl, das durch Oxo- oder Thionogruppen substituiert ist, Phenyl, Naphthyl oder $C_7$-$C_{10}$-Phenylalkyl bedeuten oder $R^4$ und $R^5$ zusammen mit dem N-Atom, an das sie gebunden sind, keinen Pyrrolidin-, Piperidin-, Morpholin- oder N-substituierten Piperazinring bilden, und mit der Massgabe, dass die Verbindungen nicht Bis[(2-thiono-5-methyl-oxazolidino)methyl]-methylamin, Bis-[(2-thiono-5-methyl-oxazolidino)methyl]isopropylamin oder Bis-[(2-thiono-thiazolidino)methyl]-methylamin bedeuten.

**17.** Verbindungen der Formeln

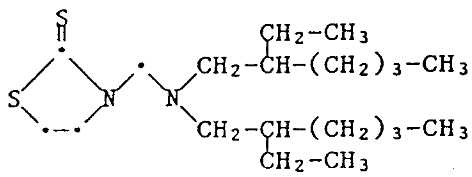 ,

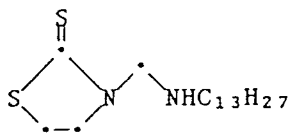 ,

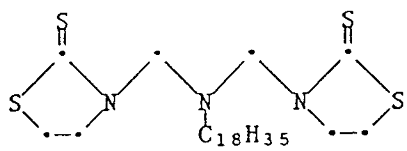 ,

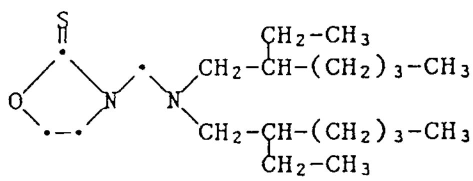 und

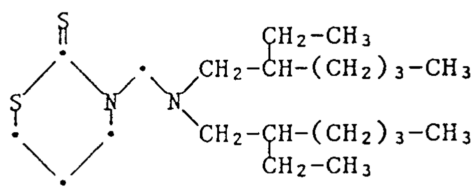 .

**Patentansprüche für folgenden Vertragsstaat : ES**

1. Zusammensetzung enthaltend ein oder mehrere Schmiermittel oder Hydrauliköle auf der Basis von Mineralöl, synthetischen Oelen oder Gemischen davon und mindestens eine Verbindung der Formeln I, II und/oder III,

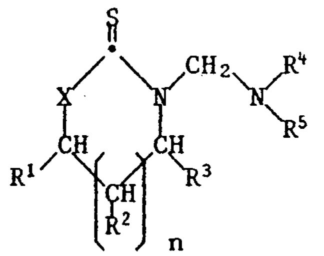

I

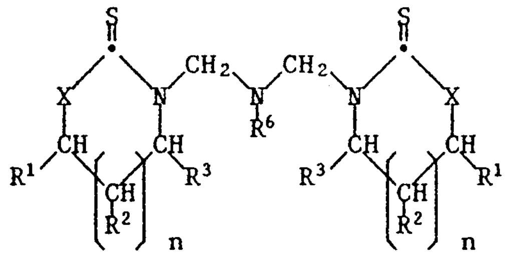

II

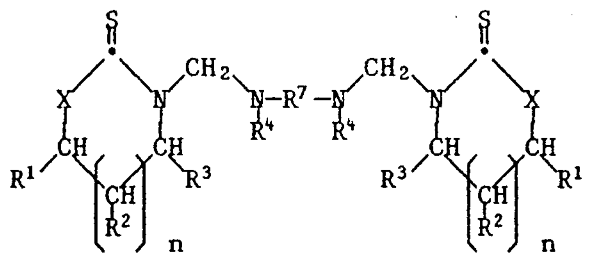

III

worin
$R^1$, $R^2$, $R^3$, R, $R^5$ und $R^6$ unabhängig voneinander $C_1$-$C_{24}$-Alkyl oder $C_1$-$C_{24}$-Alkyl das durch Oxo- oder Thionogruppen substituiert ist oder $C_3$-$C_{24}$-Alkyl, das durch

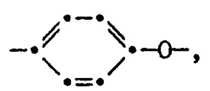

-O-, -S- und/oder -N($R^8$)-, mit $R^8$ als Wasserstoff oder $C_1$-$C_{12}$-Alkyl, unterbrochen ist oder $C_3$-$C_{24}$-Alkyl, das durch

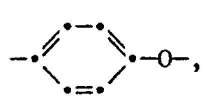

-O-, -S- und/oder -N($R^8$)-, mit $R^8$ als Wasserstoff oder $C_1$-$C_{12}$-Alkyl, unterbrochen ist und das durch Oxo-oder Thionogruppen substituiert ist, oder $C_2$-$C_{24}$-Alkenyl, oder unsubstituiertes oder durch ein oder zwei $C_1$-$C_{12}$-Alkyl-, $C_1$-$C_4$-Alkoxy-, $C_2$-$C_{24}$-Carboalkoxy- oder Nitrogruppen substituiertes Phenyl oder Naphtyl, oder $C_7$-$C_{10}$-Phenylalkyl bedeuten und $R^1$, $R^2$, $R^3$ und $R^4$ zusätzlich Wasserstoff bedeutet, oder $R^1$ und $R^2$ bzw. $R^1$ und $R^3$ im Fall von n gleich Null, zusammen mit den C-Atomen, an die sie gebunden sind, einen 5- oder 6-gliedrigen aliphatischen Ring bilden oder einen 5- oder 6-gliedrigen aliphatischen Ring, der durch Oxo- oder Thionogruppen substituiert ist, bilden oder einen 5- oder 6-gliedrigen aliphatischen Ring bilden, der durch -N($R^8$)-, mit $R^8$ in der vorstehenden Bedeutung, -O-oder -S- unterbrochen ist oder einen 5- oder 6-gliedrigen aliphatischen Ring bilden, der durch -N($R^8$)-, mit $R^8$ in der vorstehenden Bedeutung, -O-oder -S- unterbrochen und durch Oxo- oder Thionogruppen substituiert ist, oder worin $R^4$ und $R^5$ zusammen mit dem N-Atom, an das sie gebunden sind, einen 5- bis 7-gliedrigen aliphatischen-heterocyclischen Ring bilden, oder worin $R^4$ und $R^5$ zusammen mit dem N-Atom, an das sie gebunden sind, einen 5- bis 7-gliedrigen aliphatischenheterocyclischen Ring bilden und der Ring durch Oxo- oder Thionogruppen substituiert ist und/oder zusätzlich zu dem N-Atom noch

weitere Heteroatome [N($R^8$), O, S] enthält, und worin $R^7$ $C_2$-$C_{12}$-Alkylen, $C_2$-$C_{12}$-Alkylen, das durch -N($R^8$)-, -O-, und/oder -S-unterbrochen ist, $C_2$-$C_{12}$-Alkylen, das Oxo- oder Thionogruppen enthält oder $C_2$-$C_{12}$-Alkylen, das durch -N($R^8$)-, -O- und/oder -S- unterbrochen ist und das Oxo- oder Thionogruppen enthält, $C_6$-$C_{15}$-Cycloalkylen, $C_6$-$C_{15}$-Arylen, Carbonyl oder Thiocarbonyl bedeutet oder die Gruppe -N($R^4$)-$R^7$-N($R^4$)-einen Piperazin-1,4-diylrest oder einen Piperazin-1,4-diylrest, der durch eine oder mehrere Methylgruppen substituiert ist, darstellt, und X Sauerstoff oder Schwefel und n die Zahlen Null oder 1 bedeuten.

**2.** Zusammensetzung gemäss Anspruch 1, worin in den Verbindungen der Formeln I, II oder III n gleich 1 ist.

**3.** Zusammensetzung gemäss Anspruch 2, worin in den Verbindungen der Formeln I, II oder III $R^2$ Wasserstoff, $C_1$-$C_{12}$-Alkyl, Phenyl oder Benzyl bedeutet.

**4.** Zusammensetzung gemäss Anspruch 2, worin in den Verbindungen der Formeln I, II oder III $R^1$ und $R^2$ zusammen mit den C-Atomen, an die sie gebunden sind, einen 6-gliedrigen aliphatischen Ring bilden.

**5.** Zusammensetzung gemäss Anspruch 1, worin in den Verbindungen der Formeln I, II oder III n gleich Null ist.

**6.** Zusammensetzung gemäss Anspruch 1, worin in den Verbindungen der Formeln I, II oder III $R^1$ Wasserstoff, $C_1$-$C_{12}$-Alkyl, $C_1$-$C_{12}$-Alkyl das durch eine Oxogruppe substituiert ist, $C_3$-$C_{12}$-Alkyl, das durch

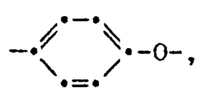

-O- oder -S- unterbrochen ist oder $C_3$-$C_{12}$-Aklyl, das durch

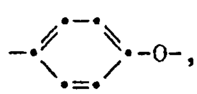

-O- oder -S- unterbrochen und durch eine Oxogruppe substituiert ist, Phenyl oder Benzyl bedeutet.

**7.** Zusammensetzung gemäss Anspruch 1, worin in den Verbindungen der Formeln I, II oder III $R^3$ Wasserstoff oder $C_1$-$C_4$-Alkyl bedeutet.

**8.** Zusammensetzung gemäss Anspruch 1, worin in den Verbindungen der Formeln I oder III $R^4$ Wasserstoff, $C_1$-$C_{12}$-Alkyl, $C_3$-$C_{18}$-Alkenyl, Phenyl oder Benzyl bedeutet.

**9.** Zusammensetzung gemäss Anspruch 1, worin in der Verbindung der Formel I $R^5$ $C_1$-$C_{18}$-Alkyl, $C_3$-$C_{18}$-Alkenyl, Phenyl oder Benzyl bedeutet.

**10.** Zusammensetzung gemäss Anspruch 1, worin in der Verbindung der Formel I $R^4$ und $R^5$ zusammen mit dem N-Atom, an das sie gebunden sind einen Piperidin-, Perhydroazepin-, Morpholin-, Piperazin- oder einen 4-N-Methylpiperazinring bilden.

**11.** Zusammensetzung gemäss Anspruch 1, worin in der Verbindung der Formel II $R^6$ $C_1$-$C_{12}$-Alkyl, $C_3$-$C_{18}$-Alkenyl, Phenyl oder Benzyl bedeutet.

**12.** Zusammensetzung gemäss Anspruch 1, worin in den Verbindungen der Formeln I, II oder III X Schwefel bedeutet.

**13.** Zusammensetzung gemäss Anspruch 1, worin die Menge der Verbindungen der Formeln I, II oder III

0,05 bis 5 Gew.-%, bezogen auf das Gesamtgewicht der Zusammensetzung, beträgt.

**14.** Zusammensetzung gemäss Anspruch 1, worin in den Verbindungen der Formeln I und II n Null, $R^1$ und $R^3$ Wasserstoff, $R^4$ und $R^5$ unabhängig voneinander $C_1$-$C_{18}$-Alkyl oder $C_3$-$C_{18}$-Alkenyl und $R^6$ $C_3$-$C_{18}$-Alkenyl und X Schwefel bedeuten.

**15.** Verwendung von mindestens einer Verbindung der Formeln I, II oder III nach Anspruch 1 als Hochdruck- oder verschleissmindernde Zusätze zu mineralischen oder synthetischen Schmiermitteln oder Hydraulikölen.

## Claims
**Claims for the following Contracting States : BE, DE, FR, GB, IT, NL**

**1.** A composition containing one or more lubricants or hydraulic oils based on mineral oil, synthetic oils or mixtures thereof and at least one compound of the formulae I, II and/or III,

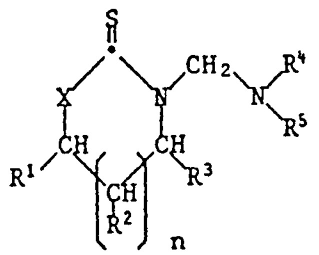

I

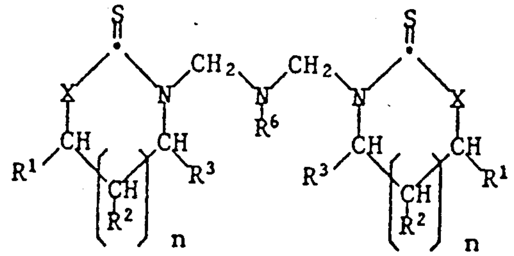

II

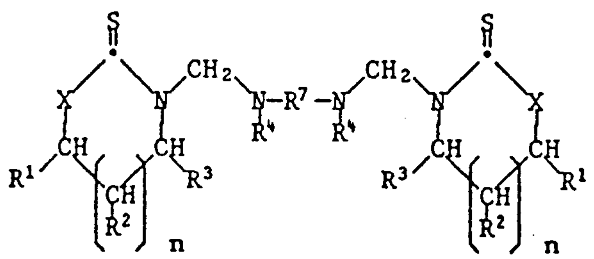

III

in which $R^1$, $R^2$, $R^3$, $R^4$, $R^5$ and $R^6$ independently of one another are $C_1$-$C_{24}$alkyl or $C_1$-$C_{24}$alkyl which is substituted by oxo or thiono groups or $C_3$-$C_{24}$alkyl which is interrupted by

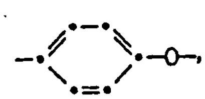

-O-, -S- and/or -N($R^8$)-in which $R^8$ is hydrogen or $C_1$-$C_{12}$alkyl, or $C_3$-$C_{24}$alkyl which is interrupted by

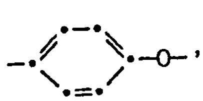

-O-, -S- and/or -N($R^8$)- in which $R^8$ is hydrogen or $C_1$-$C_{12}$alkyl, and which is substituted by oxo or thiono groups, or $C_2$-$C_{24}$alkenyl, or phenyl or naphthyl which is unsubstituted or is substituted by one or two $C_1$-$C_{12}$alkyl, $C_1$-$C_4$alkoxy, $C_2$-$C_{24}$carboalkoxy or nitro groups, or are $C_7$-$C_{10}$phenylalkyl, and $R^1$,

$R^2$, $R^3$ and $R^4$ additionally are hydrogen, or $R^1$ and $R^2$, or $R^1$ and $R^3$ in the event that n is zero, together with the C atoms to which they are attached, form a 5-membered or 6-membered aliphatic ring or form a 5-membered or 6-membered aliphatic ring which is substituted by oxo or thiono groups, or form a 5-membered or 6-membered aliphatic ring which is interrupted by $-N(R^5)$-in which $R^8$ is as defined above, -O- or -S-, or form a 5-membered or 6-membered aliphatic ring which is interrupted by $-N(R^8)$- in which $R^8$ is as defined above, -O- or -S- and is substituted by oxo or thiono groups, or in which $R^4$ and $R^5$, together with the N atom to which they are attached, form a 5-membered to 7-membered aliphatic-heterocyclic ring, or in which $R^4$ and $R^5$, together with the N atom to which they are attached, form a 5-membered to 7-membered aliphatic-heterocyclic ring and the ring is substituted by oxo or thiono groups and/or contains, additionally to the N atom, further hetero atoms [$N(R^8)$, O or S] and in which $R^7$ is $C_2$-$C_{12}$alkylene, $C_2$-$C_{22}$alkylene which is interrupted by $-N(R^8)$-, -O- and/or -S-, $C_2$-$C_{12}$alkylene which contains oxo or thiono groups or $C_2$-$C_{12}$-alkylene which is interrupted by $-N(R^8)$-, -O- and/or -S- and contains oxo or thiono groups, $C_6$-$C_{15}$cycloalkylene, $C_6$-$C_{15}$arylene, carbonyl or thiocarbonyl, or the group $-N(R^4)-R^7-N(R^4)$- is a piperazine-1,4-diyl radical or a piperazine-1,4-diyl radical which is substituted by one or more methyl groups, and X is oxygen or sulphur and n is the number zero or 1.

2. A composition according to claim 1, wherein, in the compounds of the formulae I, II and III, n is 1.

3. A composition according to claim 2, wherein, in the compounds of the formulae I, II and III, $R^2$ is hydrogen, $C_1$-$C_{12}$alkyl, phenyl or benzyl.

4. A composition according to claim 2, wherein, in the compounds of the formulae I, II and III, $R^1$ and $R^2$, together with the C atoms to which they are attached, form a 6-membered aliphatic ring.

5. A composition according to claim 1, wherein, in the compounds of the formulae I, II and III, n is zero.

6. A composition according to claim 1, wherein, in the compounds of the formulae I, II and III, $R^1$ is hydrogen, $C_1$-$C_{12}$alkyl, $C_1$-$C_{12}$alkyl which is substituted by an oxo group, $C_3$-$C_{12}$alkyl which is interrupted by

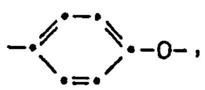

-O- or -S-, or $C_3$-$C_{12}$alkyl which is interrupted by

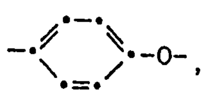

-O- or -S- and substituted by an oxo group, or is phenyl or benzyl.

7. A composition according to claim 1, wherein, in the compounds of the formulae I, II and III, $R^3$ is hydrogen or $C_1$-$C_4$alkyl.

8. A composition according to claim 1, wherein, in the compounds of the formulae I and III, $R^4$ is hydrogen, $C_1$-$C_{12}$alkyl, $C_3$-$C_{18}$alkenyl, phenyl or benzyl.

9. A composition according to claim 1, wherein, in the compound of the formula I, $R^5$ is $C_1$-$C_{18}$alkyl, $C_3$-$C_{18}$alkenyl, phenyl or benzyl.

10. A composition according to claim 1, wherein, in the compound of the formula I, $R^4$ and $R^5$, together with the N atom to which they are attached, form a piperidine, perhydroazepine, morpholine, piperazine or a 4-N-methylpiperazine ring.

11. A composition according to claim 1, wherein, in the compound of the formula II, $R^6$ is $C_1$-$C_{12}$alkyl, $C_3$-$C_{18}$alkenyl, phenyl or benzyl.

**12.** A composition according to claim 1, wherein, in the compounds of the formulae I, II and III, X is sulphur.

**13.** A composition according to claim 1, wherein the amount of the compounds of the formulae I, II and III is 0.05 to 5% by weight, relative to the total weight of the composition.

**14.** A composition according to claim 1, wherein, in the compounds of the formulae I and II, n is zero, $R^1$ and $R^3$ are hydrogen, $R^4$ and $R^5$ independently of one another are $C_1$-$C_{18}$alkyl or $C_3$-$C_{18}$alkenyl and $R^6$ is $C_3$-$C_{18}$alkenyl and X is sulphur.

**15.** The use of at least one compound of the formulae I, II and III according to claim 1 as a high-pressure additive or wear-reducing additive for mineral or synthetic lubricants or hydraulic oils.

**16.** A compound of the formula $I^*$, $II^*$ or $III^*$

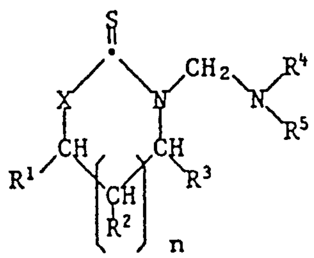

$I^*$

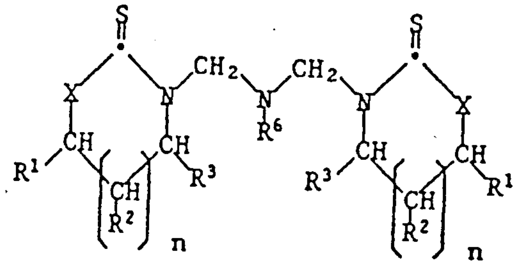

$II^*$

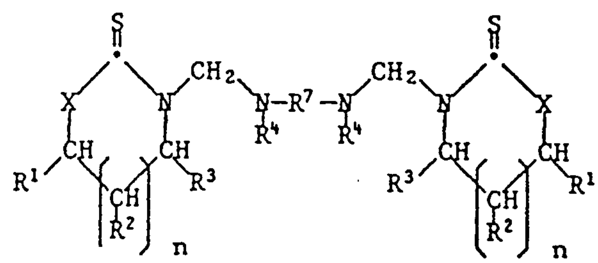

$III^*$

in which $R'$, $R^2$, $R^3$, $R^4$, $R^5$ and $R^6$ independently of one another are $C_1$-$C_{24}$alkyl or $C_1$-$C_{24}$alkyl which is substituted by oxo or thiono groups or $C_3$-$C_{24}$alkyl which is interrupted by

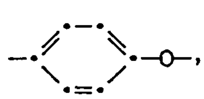

-O-, -S- and/or -N($R^8$)-in which $R^8$ is hydrogen or $C_1$-$C_{12}$alkyl, or $C_3$-$C_{24}$-alkyl which is interrupted by

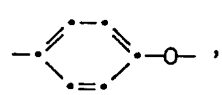

-O-, -S- and/or -N($R^8$)- in which $R^8$ is hydrogen or $C_1$-$C_{12}$alkyl, and which is substituted by oxo or thiono groups, or $C_2$-$C_{24}$alkenyl, or phenyl or naphthyl which is unsubstituted or is substituted by one or two $C_1$-$C_{12}$alkyl, $C_1$-$C_4$alkoxy, $C_2$-$C_{24}$carboalkoxy or nitro groups, or are $C_7$-$C_{10}$phenylalkyl, and $R^1$,

$R^2$, $R^3$ and $R^4$ additionally are hydrogen, or $R^1$ and $R^2$, or $R^1$ and $R^3$ in the event that n is zero, together with the C atoms to which they are attached, form a 5-membered or 6-membered aliphatic ring or form a 5-membered or 6-membered aliphatic ring which is substituted by oxo or thiono groups, or form a 5-membered or 6-membered aliphatic ring which is interrupted by -N($R^8$)-in which $R^8$ is as defined above, -O- or -S-, or form a 5-membered or 6-membered aliphatic ring which is interrupted by -N($R^8$)-in which $R^8$ is as defined above, -O- or -S- and is substituted by oxo or thiono groups, or in which $R^4$ and $R^5$, together with the N atom to which they are attached, form a 5-membered to 7-membered aliphatic-heterocyclic ring, or in which $R^4$ and $R^5$, together with the N atom to which they are attached, form a 5-membered to 7-membered aliphatic-heterocyclic ring and the ring is substituted by oxo or thiono groups and/or contains, additionally to the N atom, further hetero atoms [N($R^8$), O or S] and in which $R^7$ is $C_2$-$C_{12}$-alkylene, $C_2$-$C_{12}$alkylene which is interrupted by -N($R^8$)-, -O- and/or -S-, $C_2$-$C_{12}$alkylene which contains oxo or thiono groups or $C_2$-$C_{12}$-alkylene which is interrupted by -N($R^8$)-, -O- and/or -S-and contains oxo or thiono groups, $C_6$-$C_{15}$cycloalkylene, $C_6$-$C_{15}$arylene, carbonyl or thiocarbonyl, or the group -N($R^4$)-$R^7$-N($R^4$)- is a piperazine-1,4-diyl radical or a piperazine-1,4-diyl radical which is substituted by one or more methyl groups, and X is oxygen or sulphur and n is the number zero or 1, where, in the event that $R^1$ and $R^3$ are hydrogen, $C_1$-$C_{24}$alkyl, $C_1$-$C_{24}$alkyl which is substituted by oxo or thiono groups, $C_7$-$C_{10}$phenylalkyl or naphthyl or phenyl unsubstituted or substituted by one or two $C_1$-$C_{12}$alkyl, $C_1$-$C_4$alkoxy, $C_2$-$C_{24}$carboalkoxy or nitro groups, then $R^4$ is not hydrogen and $R^4$ and $R^5$ are not $C_1$-$C_{24}$alkyl, $C_1$-$C_{24}$alkyl which is substituted by oxo or thiono groups, phenyl, naphthyl or $C_7$-$C_{10}$phenylalkyl, nor do $R^4$ and $R^5$, together with the N atom to which they are attached, form a pyrrolidine, piperidine, morpholine or N-substituted piperazine ring, and subject to the proviso that the compounds are not bis-[(2-thiono-5-methyloxazolidino)-methyl]-methylamine, bis-[(2-thiono-5-methyloxazolidino)methyl]-isopropylamine or bis[(2-thionothiazolidino)methyl]-methylamine.

**17.** A compound of the formula

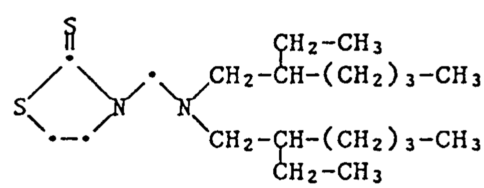

,

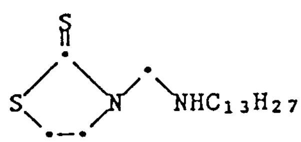

,

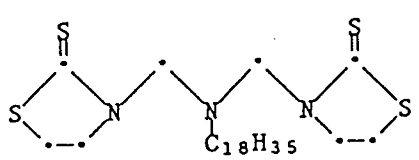

,

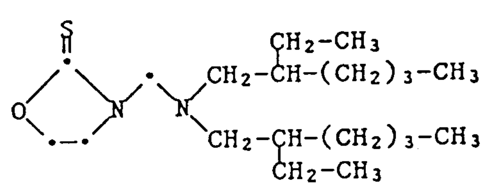

or

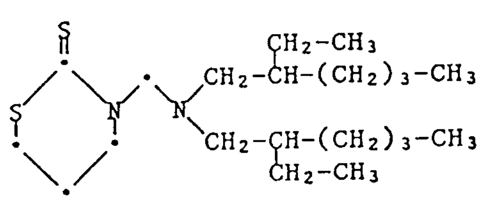

.

**Claims for the following Contracting State : ES**

**1.** A composition containing one or more lubricants or hydraulic oils based on mineral oil, synthetic oils or mixtures thereof and at least one compound of the formulae I, II and/or III,

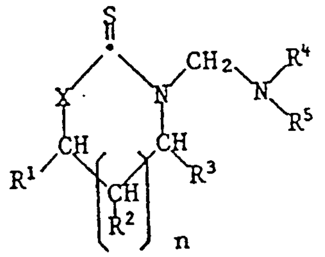

I

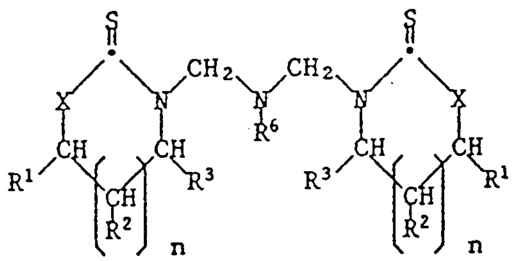

II

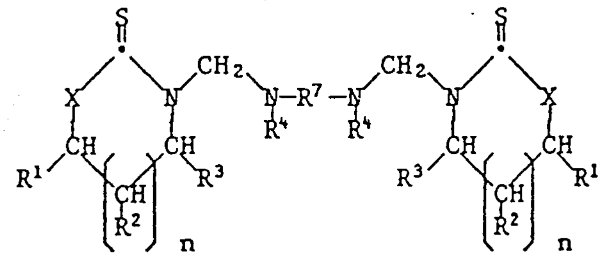

III

in which $R^1$, $R^2$, $R^3$, $R^4$, $R^5$ and $R^6$ independently of one another are $C_1$-$C_{24}$alkyl or $C_1$-$C_{24}$alkyl which is substituted by oxo or thiono groups or $C_3$-$C_{24}$alkyl which is interrupted by

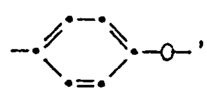

-O-, -S- and/or -N($R^8$)-in which $R^8$ is hydrogen or $C_1$-$C_{12}$alkyl, or $C_3$-$C_{24}$alkyl which is interrupted by

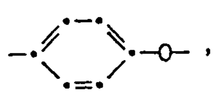

-O-, -S- and/or -H($R^8$)- in which $R^8$ is hydrogen or $C_1$-$C_{12}$alkyl, and which is substituted by oxo or thiono groups, or $C_2$-$C_{24}$alkenyl, or phenyl or naphthyl which is unsubstituted or is substituted by one or two $C_1$-$C_{12}$alkyl, $C_1$-$C_4$alkoxy, $C_2$-$C_{24}$carboalkoxy or nitro groups, or are $C_7$-$C_{10}$phenylalkyl, and R', $R^2$, $R^3$ and $R^4$ additionally are hydrogen, or $R^1$ and $R^2$, or R' and $R^3$ in the event that n is zero, together with the C atoms to which they are attached, form a 5-membered or 6-membered aliphatic ring or form a 5-membered or 6-membered aliphatic ring which is substituted by oxo or thiono groups, or form a 5-membered or 6-membered aliphatic ring which is interrupted by -N($R^8$)-in which $R^8$ is as defined above, -O- or -S-, or form a 5-membered or 6-membered aliphatic ring which is interrupted by -N($R^8$)-in which $R^8$ is as defined above, -O- or -S- and is substituted by oxo or thiono groups, or in which $R^4$ and $R^5$, together with the N atom to which they are attached, form a 5-membered to 7-membered aliphatic-heterocyclic ring, or in which $R^4$ and $R^5$, together with the N atom to which they are attached, form a 5-membered to 7-membered aliphatic-heterocyclic ring and the ring is substituted by oxo or thiono groups and/or contains, additionally to the N atom, further hetero atoms [N($R^8$), O or S] and in

which $R^7$ is $C_2$-$C_{12}$alkylene, $C_2$-$C_{12}$alkylene which is interrupted by -N($R^8$)-, -O- and/or -S-, $C_2$-$C_{12}$alkylene which contains oxo or thiono groups or $C_2$-$C_{12}$-alkylene which is interrupted by -N($R^8$)-, -O- and/or -S- and contains oxo or thiono groups, $C_6$-$C_{15}$cycloalkylene, $C_6$-$C_{15}$arylene, carbonyl or thiocarbonyl, or the group -N($R^4$)-$R^7$-N($R^4$)- is a piperazine-1,4-diyl radical or a piperazine-1,4-diyl radical which is substituted by one or more methyl groups, and X is oxygen or sulphur and n is the number zero or 1.

**2.** A composition according to claim 1, wherein, in the compounds of the formulae I, II and III, n is 1.

**3.** A composition according to claim 2, wherein, in the compounds of the formulae I, II and III, $R^2$ is hydrogen, $C_1$-$C_{12}$alkyl, phenyl or benzyl.

**4.** A composition according to claim 2, wherein, in the compounds of the formulae I, II and III, $R^1$ and $R^2$, together with the C atoms to which they are attached, form a 6-membered aliphatic ring.

**5.** A composition according to claim 1, wherein, in the compounds of the formulae I, II and III, n is zero.

**6.** A composition according to claim 1, wherein, in the compounds of the formulae I, II and III, $R^1$ is hydrogen, $C_1$-$C_{12}$alkyl, $C_1$-$C_{12}$alkyl which is substituted by an oxo group, $C_3$-$C_{12}$alkyl which is interrupted by

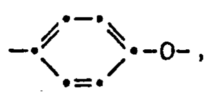

-O- or -S-, or $C_3$-$C_{12}$alkyl which is interrupted by

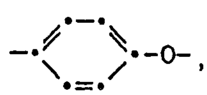

-O- or -S- and substituted by an oxo group, or is phenyl or benzyl.

**7.** A composition according to claim 1, wherein, in the compounds of the formulae I, II and III, $R^3$ is hydrogen or $C_1$-$C_4$alkyl.

**8.** A composition according to claim 1, wherein, in the compounds of the formulae I and III, $R^4$ is hydrogen, $C_1$-$C_{12}$alkyl, $C_3$-$C_{18}$alkenyl, phenyl or benzyl.

**9.** A composition according to claim 1, wherein, in the compound of the formula I, $R^5$ is $C_1$-$C_{18}$alkyl, $C_3$-$C_{18}$alkenyl, phenyl or benzyl.

**10.** A composition according to claim 1, wherein, in the compound of the formula I, $R^4$ and $R^5$, together with the N atom to which they are attached, form a piperidine, perhydroazepine, morpholine, piperazine or a 4-N-methylpiperazine ring.

**11.** A composition according to claim 1, wherein, in the compound of the formula II, $R^6$ is $C_1$-$C_{12}$alkyl, $C_3$-$C_{18}$alkenyl, phenyl or benzyl.

**12.** A composition according to claim 1, wherein, in the compounds of the formulae I, II and III, X is sulphur.

**13.** A composition according to claim 1, wherein the amount of the compounds of the formulae I, II and III is 0.05 to 5% by weight, relative to the total weight of the composition.

**14.** A composition according to claim 1, wherein, in the compounds of the formulae I and II, n is zero, $R^1$ and $R^3$ are hydrogen, $R^4$ and $R^5$ independently of one another are $C_1$-$C_{18}$alkyl or $C_3$-$C_{18}$alkenyl and $R^6$ is $C_3$-$C_{18}$alkenyl and X is sulphur.

**15.** The use of at least one compound of the formulae I, II and III according to claim 1 as a high-pressure additive or wear-reducing additive for mineral or synthetic lubricants or hydraulic oils.

## Revendications

## Revendications pour les Etats contractants suivants : BE, DE, FR, GB, IT, NL

**1.** Composition comprenant un ou plusieurs lubrifiants ou huiles hydrauliques à base d'une huile minérale, d'huiles synthétiques ou de mélanges de ces huiles, avec un ou plusieurs composés de formules I, II ou III ci-dessous.

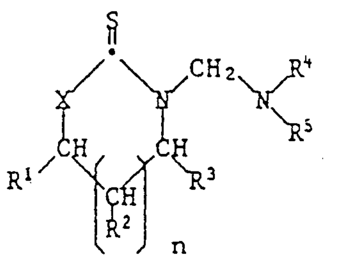

I

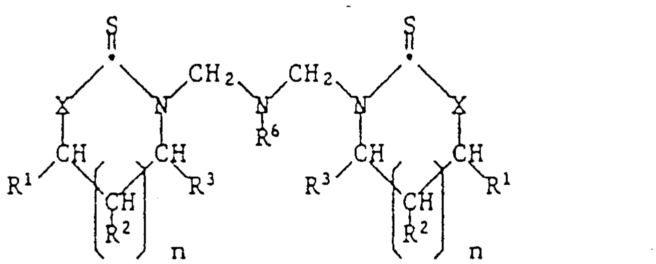

II

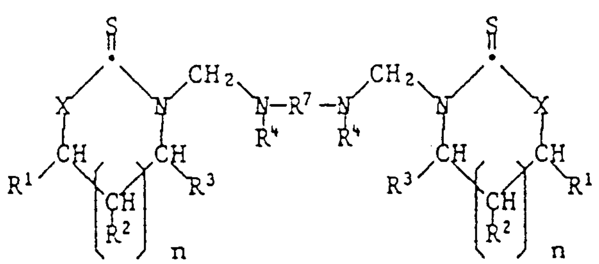

III

formules dans lesquelles :
$R^1$, $R^2$, $R^3$, $R^4$, $R^5$ et $R^6$ représentent chacun, indépendamment les uns des autres, un $C_1$-$C_{24}$-alkyle avec éventuellement des groupes oxo ou thiono ou bien un $C_3$-$C_{24}$-alkyle interrompu par

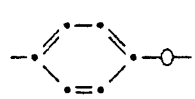 ,

-O-, -S- et/ou -N($R^8$)-, $R^8$ désignant l'hydrogène ou un alkyle en $C_1$-$C_{12}$, ou à la fois interrompu par

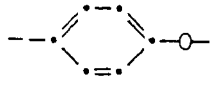 ,

-O-, -S- et/ou -N($R^8$)-, $R^8$ désignant l'hydrogène ou un alkyle en $C_1$-$C_{12}$, et substitué par des groupes oxo ou thiono, ou encore un $C_2$-$C_{24}$-alcényle, un phényle ou un naphtyle sans substituants ou avec un ou deux substituants pouvant être des alkyles en $C_1$-$C_{12}$, des alcoxy en $C_1$-$C_4$, des alcoxycarbonyles en $C_2$-$C_{24}$ ou des groupes nitro, ou un phénylalkyle en $C_7$-$C_{10}$, $R^1$, $R^2$, $R^3$ et $R^4$ pouvant représenter en

outre l'hydrogène, ou bien $R^1$ et $R^2$ ou $R^1$ et $R^3$, dans le cas où le nombre n est nul, forment ensemble et avec les atomes de carbone auxquels ils sont liés un cycle aliphatique pentagonal ou hexagonal éventuellement avec des groupes oxo ou thiono ou interrompu par un groupe $-N(R^8)-$, $R^8$ ayant la signification ci-dessus, de l'oxygène ou du soufre, ou encore pouvant être à la fois interrompu par un groupe $-N(R^8)-$, de l'oxygène ou du soufre et substitué par des groupes oxo ou thiono, ou dans lesquelles $R^4$ et $R^5$ forment ensemble et avec l'atome d'azote auquel ils sont liés un cycle aliphatique-hétérocyclique pentagonal à heptagonal avec éventuellement des groupes oxo ou thiono et/ou en outre encore d'autres hétéroatomes [$N(R^8)$, O, S], et $R^7$ représente un $C_2$-$C_{12}$-alkylène, éventuellement interrompu par un groupe $-N(R^8)-$, un atome d'oxygène et/ou un atome de soufre ou comportant éventuellement des groupes oxo ou thiono, ou encore un $C_2$-$C_{12}$-alkylène à la fois interrompu par un groupe $(N)-R^8-$, un atome d'oxygène et/ou de soufre et comportant des groupes oxo ou thiono, un $C_6$-$C_{15}$-cycloalkylène, un $C_6$-$C_{15}$-arylène, un groupe carbonyle ou thiocarbonyle, ou encore le groupe $-N-(R^4)-R^7-N-(R^4)-$, ou un radical pipérazine-1,4-diyle pouvant avoir éventuellement un ou plusieurs groupes méthyliques, X désigne un atome d'oxygène ou de soufre et n est le nombre 0 ou 1.

**2.** Composition selon la revendication 1 dans laquelle, dans les composés de formule I, II ou III, n est le nombre 1.

**3.** Composition selon la revendication 2 dans laquelle, dans les composés de formule I, II ou III, $R^2$ est l'hydrogène, un alkyle en $C_1$-$C_{12}$, un phényle ou un benzyle.

**4.** Composition selon la revendication 2 dans laquelle, dans les composés de formule I, II ou III, $R^1$ et $R^2$ forment ensemble et avec les atomes de carbone auxquels ils sont liés un cycle aliphatique hexagonal.

**5.** Composition selon la revendication 1 dans laquelle, dans les composés de formule I, II ou III, n est le nombre 0.

**6.** Composition selon la revendication 1 dans laquelle, dans les composés de formule I, II ou III $R^1$ est l'hydrogène, un alkyle en $C_1$-$C_{12}$ pouvant éventuellement avoir un groupe oxo, un alkyle en $C_3$-$C_{12}$ interrompu par

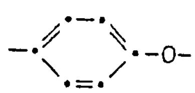 ,

-O- ou -S- ou bien à la fois interrompu par

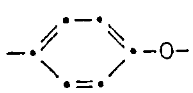 ,

-O- ou -S- et portant un groupe oxo, ou bien un phényle ou un benzyle.

**7.** Composition selon la revendication 1 dans laquelle, dans les composés de formule I, II ou III, $R^3$ est l'hygrodène ou un alkyle en $C_1$-$C_4$.

**8.** Composition selon la revendication 1 dans laquelle, dans les composés de formule I ou III, $R^4$ est l'hygrodène, un alkyle en $C_1$-$C_{12}$, un alcényle en $C_3$-$C_{18}$, un phényle ou un benzyle.

**9.** Composition selon la revendication 1 dans laquelle, dans les composés de formule I, $R^5$ est un alkyle en $C_1$-$C_{18}$, un alcényle en $C_3$-$C_{18}$, un phényle ou un benzyle.

**10.** Composition selon la revendication 1 dans laquelle, dans les composés de formule I, $R^4$ et $R^5$ forment ensemble et avec l'atome d'azote auquel ils sont liés un cycle de pipéridine, de perhydroazépine, de morpholine, de pipérazine ou de 4-N-méthylpipérazine.

**11.** Composition selon la revendication 1 dans laquelle, dans les composés de formule II, $R^6$ est un alkyle

en $C_1$-$C_{12}$, un alcényle en $C_3$-$C_{18}$, un phényle ou un benzyle.

**12.** Composition selon la revendication 1 dans laquelle, dans les composés de formule I, II ou III, X est un atome de soufre.

**13.** Composition selon la revendication 1 dont la proportion des composés de formule I, II ou III est de 0,05 à 5 % du poids total de la composition.

**14.** Composition selon la revendication 1 dans laquelle, dans les composés de formules I et II, n = 0, $R^1$ et $R^3$ sont l'hygrodène, $R^4$ et $R^5$ sont chacun, indépendamment l'un de l'autre, un alkyle en $C_1$-$C_{18}$ ou un alcényle en $C_3$-$C_{18}$, $R^6$ est un alcényle en $C_3$-$C_{18}$ et X un atome de soufre.

**15.** L'emploi des composés de formules I, II et/ou III selon la revendication 1 comme additifs pour hautes pressions ou destinés à diminuer l'usure par abrasion dans des lubrifiants ou huiles hydrauliques minéraux ou synthétiques.

**16.** Les composés de formule I*, II* ou III*.

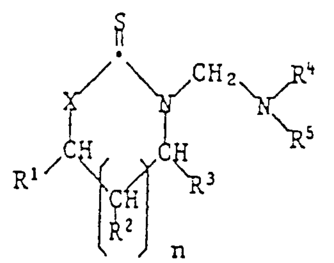

I*

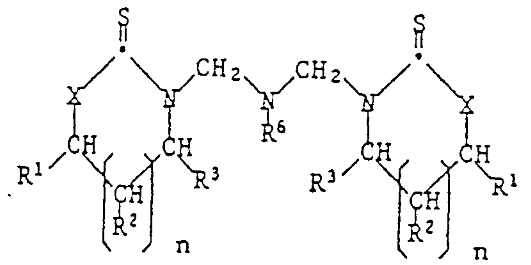

II*

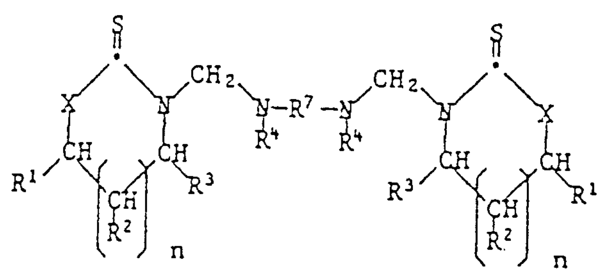

III*

formules dans lesquelles :
$R^1$, $R^2$, $R^3$, $R^4$, $R^5$ et $R^6$ représentent chacun, indépendamment les uns des autres, un $C_1$-$C_{24}$-alkyle avec éventuellement des groupes oxo ou thiono ou bien un $C_3$-$C_{24}$-alkyle interrompu par

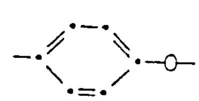 ,

-O-, -S- et/ou -N($R^8$)-, $R^8$ désignant l'hydrogène ou un alkyle en $C_1$-$C_{12}$, ou à la fois interrompu par

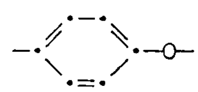

-O-, -S- et/ou -N($R^8$)-, $R^8$ désignant l'hydrogène ou un alkyle en $C_1$-$C_{12}$, et substitué par des groupes oxo ou thiono, ou encore un $C_2$-$C_{24}$-alcényle, un phényle ou un naphtyle sans substituants ou avec un ou deux substituants pouvant être des alkyles en $C_1$-$C_{12}$, des alcoxy en $C_1$-$C_4$, des alcoxycarbonyles en $C_2$-$C_{24}$ ou des groupes nitro, ou un phénylalkyle en $C_7$-$C_{10}$, $R^1$, $R^2$, $R^3$ et $R^4$ pouvant représenter en outre l'hydrogène, ou bien $R^1$ et $R^2$ ou $R^1$ et $R^3$, dans le cas où le nombre n est nul, forment ensemble et avec les atomes de carbone auxquels ils sont liés un cycle aliphatique pentagonal ou hexagonal éventuellement avec des groupes oxo ou thiono ou interrompu par un groupe -N($R^8$)-, $R^8$ ayant la signification ci-dessus, de l'oxygène ou du soufre, ou encore pouvant être à la fois interrompu par un groupe -N($R^8$)-, de l'oxygène ou du soufre et substitué par des groupes oxo ou thiono, ou dans lesquelles $R^4$ et $R^5$ forment ensemble et avec l'atome d'azote auquel ils sont liés un cycle aliphatique-hétérocyclique pentagonal à heptagonal avec éventuellement des groupes oxo ou thiono et/ou en outre encore d'autres hétéroatomes [N($R^8$), O, S] que l'atome d'azote, et $R^7$ représente un $C_2$-$C_{12}$-alkylène, éventuellement interrompu par un groupe -N($R^8$)-, un atome d'oxygène et/ou un atome de soufre ou comportant éventuellement des groupes oxo ou thiono, ou encore un $C_2$-$C_{12}$-alkylène à la fois interrompu par un groupe (N)-$R^8$-, un atome d'oxygène et/ou de soufre et comportant des groupes oxo ou thiono, un $C_6$-$C_{15}$-cycloalkylène, un $C_6$-$C_{15}$-arylène, un groupe carbonyle ou thiocarbonyle, ou encore le groupe -N($R^4$)-$R^7$-N-($R^4$)-, ou un radical pipérazine-1,4-diyle pouvant avoir éventuellement un ou plusieurs groupes méthyliques, X désigne un atome d'oxygène ou de soufre et n est le nombre 0 ou 1, mais si $R^1$ et $R^3$ sont l'hydrogène, des $C_1$-$C_{24}$-alkyles avec éventuellement des groupes oxo ou thiono, un phényle ou un naphtyle sans substituants ou avec comme substituants un ou deux $C_1$-$C_{12}$-alkyles, $C_1$-$C_4$-alcoxy, $C_2$-$C_{24}$-alcoxycarbonyles ou groupes nitro, ou encore un $C_7$-$C_{10}$-phénylalkyle, $R^4$ n'est pas l'hydrogène et $R^4$ et $R^5$ ne sont pas des $C_1$-$C_{24}$-alkyles avec éventuellement des groupes oxo ou thiono, ni un phényle, un naphtyle ou $C_7$-$C_{10}$-phénylalkyle, ou encore R4 et R5 ne forment pas avec l'atome d'azote auquel ils sont liés un cycle de pyrrolidine, de pipéridine, de morpholine ou de pipérazine substituée sur l'azote, et encore avec la condition que ces composés ne soient ni la bis-[(2-thiono-5-méthyl-oxazolidino)méthyl]-méthylamine, ni la bis-[(2-thiono-5-méthyl-oxazolidino)méthyl]-iso-propylamine, ni la bis-[(2-thiono-thiazolidine)méthyl]-méthylamine.

**17.** Les composés de formules.

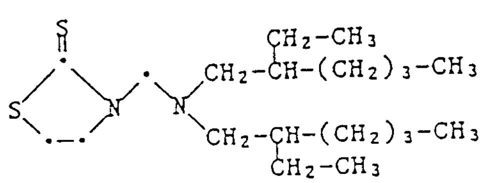

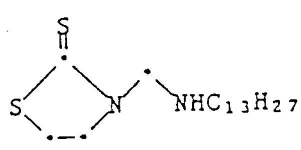

,

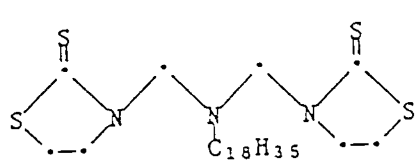

,

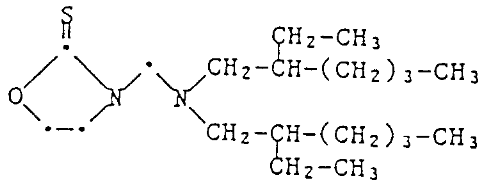

et

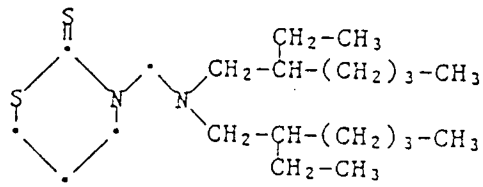

**Revendications pour l'Etat contractant suivant : ES**

**1.** Composition comprenant un ou plusieurs lubrifiants ou huiles hydrauliques à base d'une huile minérale, d'huiles synthétiques ou de mélanges de ces huiles, avec un ou plusieurs composés de formules I, II ou III ci-dessous.

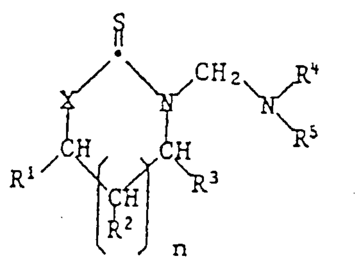

I

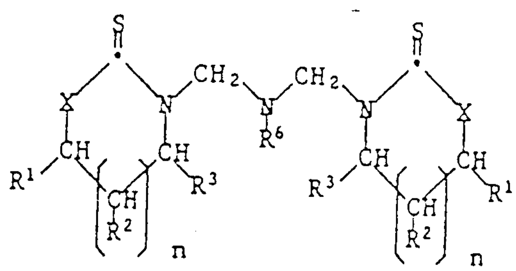

II

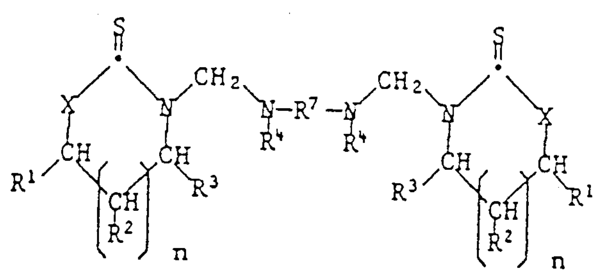

III

formules dans lesquelles :
$R^1{}_1$ $R^2$, $R^3$, $R^4$, $R^5$ et $R^6$ représentent chacun, indépendamment les uns des autres, un $C_1$-$C_{24}$-alkyle avec éventuellement des groupes oxo ou thiono ou bien un $C_3$-$C_{24}$-alkyle interrompu par

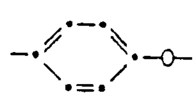 ,

-O-, -S- et/ou -N($R^8$)-, $R^8$ désignant l'hydrogène ou un alkyle en $C_1$-$C_{12}$, ou à la fois interrompu par

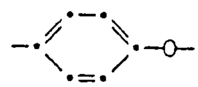 ,

-O-, -S- et/ou -N($R^8$)-, $R^8$ désignant l'hydrogène ou un alkyle en $C_1$-$C_{12}$, et substitué par des groupes oxo ou thiono, ou encore un $C_2$-$C_{24}$-alcényle, un phényle ou un naphtyle sans substituants ou avec un ou deux substituants pouvant être des alkyles en $C_1$-$C_{12}$, des alcoxy en $C_1$-$C_4$, des alcoxycarbonyles en $C_2$-$C_{24}$ ou des groupes nitro, ou un phénylalkyle en $C_7$-$C_{10}$, $R^1{}_1$ $R^2$, $R^3$ et $R^4$ pouvant représenter en outre l'hydrogène, ou bien $R^1$ et $R^2$, ou $R^1$ et $R^3$, dans le cas où le nombre n est nul, forment ensemble et avec les atomes de carbone auxquels ils sont liés un cycle aliphatique pentagonal ou hexagonal éventuellement avec des groupes oxo ou thiono ou interrompu par un groupe -N($R^8$)-, $R^8$ ayant la signification ci-dessus, de l'oxygène ou du soufre, ou encore pouvant être à la fois interrompu par un groupe -N($R^8$)-, de l'oxygène ou du soufre et substitué par des groupes oxo ou thiono, ou dans lesquelles $R^4$ et $R^5$ forment ensemble et avec l'atome d'azote auquel ils sont liés un cycle aliphatique-hétérocyclique pentagonal à heptagonal avec éventuellement des groupes oxo ou thiono et/ou en outre encore d'autres hétéroatomes [N($R^8$), O, S] et $R^7$ représente un $C_2$-$C_{12}$-alkylène, éventuellement interrompu par un groupe -N($R^8$)-, un atome d'oxygène et/ou un atome de soufre ou comportant éventuellement des groupes oxo ou thiono, ou encore un $C_2$-$C_{12}$-alkylène à la fois interrompu par un groupe (N) -$R^8$-, un atome d'oxygène et/ou de soufre et comportant des groupes oxo ou thiono, un $C_6$-$C_{15}$-cycloalkylène, un $C_6$-$C_{15}$-arylène, un groupe carbonyle ou thiocarbonyle, ou encore le groupe -N-($R^4$)-$R^7$-N-($R^4$)-, ou un radical pipérazine-1,4-diyle pouvant avoir éventuellement un ou plusieurs groupes méthyliques, X désigne un atome d'oxygène ou de soufre et n est le nombre 0 ou 1.

**2.** Composition selon la revendication 1 dans laquelle, dans les composés de formule I, II ou III, n est le nombre 1.

**3.** Composition selon la revendication 2 dans laquelle, dans les composés de formule I, II ou III, $R^2$ est l'hydrogène, un alkyle en $C_1$-$C_{12}$, un phényle ou un benzyle.

**4.** Composition selon la revendication 2 dans laquelle, dans les composés de formule I, II ou III, $R^1$ et $R^2$ forment ensemble et avec les atomes de carbone auxquels ils sont liés un cycle aliphatique hexagonal.

**5.** Composition selon la revendication 1 dans laquelle, dans les composés de formule I, II ou III, n est le nombre 0.

**6.** Composition selon la revendication 1 dans laquelle, dans les composés de formule I, II ou III $R^1$ est l'hydrogène, un alkyle en $C_1$-$C_{12}$ pouvant éventuellement avoir un groupe oxo, un alkyle en $C_3$-$C_{12}$ interrompu par :

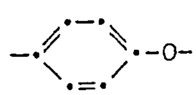

-O- ou -S- ou bien à la fois interrompu par

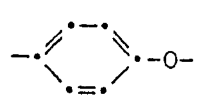

-O- ou -S- et portant un groupe oxo, ou bien un phényle ou un benzyle.

**7.** Composition selon la revendication 1 dans laquelle, dans les composés de formule I, II ou III, $R^3$ est l'hygrodène ou un alkyle en $C_1$-$C_4$.

**8.** Composition selon la revendication 1 dans laquelle, dans les composés de formule I ou III, $R^4$ est

l'hygrodène, un alkyle en $C_1$-$C_{12}$, un alcényle en $C_3$-$C_{18}$, un phényle ou un benzyle.

**9.** Composition selon la revendication 1 dans laquelle, dans les composés de formule I, $R^5$ est un alkyle en $C_1$-$C_{18}$, un alcényle en $C_3$-$C_{18}$, un phényle ou un benzyle.

**10.** Composition selon la revendication 1 dans laquelle, dans les composés de formule I, $R^4$ et $R^5$ forment ensemble et avec l'atome d'azote auquel ils sont liés un cycle de pipéridine, de perhydroazépine, de morpholine, de pipérazine ou de 4-N-méthylpipérazine.

**11.** Composition selon la revendication 1 dans laquelle, dans les composés de formule II, $R^6$ est un alkyle en $C_1$-$C_{12}$, un alcényle en $C_3$-$C_{18}$, un phényle ou un benzyle.

**12.** Composition selon la revendication 1 dans laquelle, dans les composés de formule I, II ou III, X est un atome de soufre.

**13.** Composition selon la revendication 1 dont la proportion des composés de formule I, II ou III est de 0,05 à 5 % du poids total de la composition.

**14.** Composition selon la revendication 1 dans laquelle, dans les composés de formules I et II, n = 0, $R^1$ et $R^3$ sont l'hygrodène, $R^4$ et $R^5$ sont chacun, indépendamment l'un de l'autre, un alkyle en $C_1$-$C_{18}$ ou un alcényle en $C_3$-$C_{18}$, $R^6$ est un alcényle en $C_3$-$C_{18}$ et X un atome de soufre.

**15.** L'emploi des composés de formules I, II et/ou III selon la revendication 1 comme additifs pour hautes pressions ou destinés à diminuer l'usure par abrasion dans des lubrifiants ou huiles hydrauliques minéraux ou synthétiques.